# EUROPÄISCHE PATENTSCHRIFT

(11) **EP 3 670 494 B1**
(45) Veröffentlichungstag und Bekanntmachung des Hinweises auf die Patenterteilung: **14.06.2023**
(21) Anmeldenummer: 18213331.4
(22) Anmeldetag: 18.12.2018
(51) Int. Cl.: C07C 229/76, C07F 3/00

(54) **HERSTELLUNG EINER LYSINAT-VERBINDUNG AUS EINER WÄSSRIGEN LYSINLÖSUNG**
PRODUCTION OF LYSINATE COMPOUND FROM AN AQUEOUS LYSIN SOLUTION
PRODUCTION D'UN COMPOSÉ DE LYSINATE À PARTIR D'UNE SOLUTION AQUEUSE DE LYSINE

(43) Veröffentlichungstag der Anmeldung: 24.06.2020
(73) Patentinhaber: Phytobiotics Futterzusatzstoffe GmbH, 65343 Eltville (DE)
(72) Erfinder: ROTH, Hermann, 65346 Eltville (DE); LINK, Yvonne, 65375 Oestrich-Winkel (DE); DOHMS, Juliane, 65343 Eltville (DE)
(74) Vertreter: Richardt Patentanwälte PartG mbB

(56) Entgegenhaltungen:
- EP-A1- 1 529 775
- CN-A- 104 739 816
- US-A- 4 948 594
- US-A- 5 061 815
- US-A1- 2004 137 108
- US-A1- 2010 056 817
- US-A1- 2012 225 161
- US-A1- 2018 162 803
- , Retrieved from the Internet: URL:http://www 1 .biologie.uni-hamburg.de/b-online/library/ newton/Chv251_253/Lectures/AminoAcids/Amin oAcids.html#:-:text=Lysine%20contains%20a% 20carboxylic%20acid,of%20these%20groups%20 are%20protonated.&text=This%20means%20that %20when%20the,in%201Lysine%20will%20be%20d eprotonated. [retrieved on 2021-05-06]
- THERMOCHIMICA ACTA, vol. 362, 1 January 2000 (2000-01-01), pages 13-23,
- D10A: Machine translation of D10

## Beschreibung

### Technisches Gebiet

Die Erfindung bezieht sich auf ein Verfahren zur Erzeugung von Aminosäure-Metallsalz-Verbindungen, insbesondere von Monolysinat-Verbindungen, und deren Verwendung.

### Stand der Technik

Wenn Metallverbindungen mit Aminosäuren zur Reaktion gebracht werden, entstehen Chelate oder Salzverbindungen. Aminosäure-Chelat-Verbindungen werden unter anderem in der Tierfütterung zur Versorgung mit Spurenelementen verwendet. Die derzeit im Markt erhältlichen Aminosäure-Chelat-Verbindungen unterscheiden sich hinsichtlich des Spurenelement-Gehalts, des stöchiometrischen Aminosäure-Metall-Verhältnisses, der Löslichkeit in Wasser, der Farbe und Struktur, des pH-Wertes und der Menge und Art der herstellungsbedingten Rückstände. Insbesondere der Abrieb von Mühlen, die zur mechanischen Aktivierung der Reaktanden verwendet werden, und/oder anorganischen Anionen (insb. Chloride), die in den Edukten der Reaktion enthalten sein können, stellen eine unerwünschte Quelle von Verunreinigungen dar.

Die EP 1 529775 B1 beschreibt ein Verfahren zur Herstellung von Chelaten von Metallen mit organischen Säuren, welches im Wesentlichen im wasserfreien Medium arbeitet. Der beschriebene Herstellungsprozess bedarf jedoch einer aufwändigen Vorbehandlung der eingesetzten Metallverbindungen.

Das Patent DE 10 2011 011 924 84 beschreibt ein Verfahren zur Herstellung von Aminosäure-Chelat-Verbindungen, gemäß welchem Metalloxide, Metallcarbonate, Metallsulfate, Metallchloride und/oder Metallhydroxide in fester Form durch Schlag und Druck mechanisch aktiviert werden und danach die aktivierten Metalloxide/Metallcarbonate/Metallhydroxide/Metallsulfate und/oder Metallchloride mit Aminosäuren in fester Form zusammengebracht und in einer Festkörperreaktion zu Aminosäure-Chelat-Verbindungen umgesetzt werden. Die Patentanmeldungen CN 92107282.1 und CN 2007/130121.0 beschreiben die Verwendung einer Kugelmühle zur mechanischen Aktivierung und Umsetzung von Gemischen aus Kupferacetat und Glycin zur Erzeugung von Glycin-Chelaten. Ein Nachteil beim Einsatz von Kugel- und anderen Mühlen (z.B. Schwingmühle, Rührwerkskugelmühle, Trommelmühle) ist jedoch, dass aufgrund der hohen mechanischen Beanspruchung der Mühle Abrieb entsteht, der als Verunreinigung im Aminosäure-Chelat verbleibt und ggf. mühsam aus diesem entfernt werden muss. Außerdem gibt es verfahrenstechnische Probleme, da die Reaktionsteilnehmer dazu neigen, mit festen Oberflächen (der Mühlen) zu verbacken und die Reaktion dadurch zu beeinträchtigen.

Viele der beschriebenen Verfahren zur Herstellung von Aminosäure-Chelat-Verbindungen beziehen sich zudem auf die Aminosäure Glycin, nicht auf Lysin. Lysin ist eine essentielle Aminosäure, die vom Menschen und anderen Säugetieren nicht selbst hergestellt werden kann. Lysin ist einer der wichtigsten Bausteine des Bindegewebes, vor allem des Kollagens. Daher führt Lysinmangel zu Bindegewebsschwäche. Bekannte Verfahren zur Erzeugung von Glycin-Chelaten können jedoch nicht einfach auf Lysin übertragen werden, da Glycin die kleinste und einfachste der natürlich vorkommenden Aminosäuren ist, wohingegen Lysin einen langen, kettenförmigen Aminosäurerest aufweist. Dieser Größenunterschied hat erhebliche sterische Auswirkungen auf die Chelat-Gitterstruktur und damit auch die Art der Chelat-Verbindungen, die die jeweiligen Aminosäuren eingehen können, und hat außerdem Auswirkungen auf die erforderlichen Reaktionsbedingungen. Die für Glycin beschriebenen Herstellungsverfahren sind daher nicht auf Lysin übertragbar.

### Technisches Problem und grundlegende Lösungen

Der Erfindung liegt demgemäß die Aufgabe zugrunde, ein alternatives bzw. verbesserte Verfahren zur Herstellung von Aminosäure-Metall-Verbindungen für die Aminosäure Lysin bereitzustellen, sowie eine entsprechende Verbindung.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen und Beispiele sind frei miteinander kombinierbar, sofern sie sich nicht gegenseitig ausschließen.

In einem Aspekt betrifft die Erfindung ein Verfahren zur Herstellung einer Monolysinat-Verbindung gemäß Patentanspruch 1.

Die Herstellung von Monolysinat-Verbindungen aus einer flüssigen (insb. Wässrigen) Lysinlösung kann vorteilhaft sein, da das in diesem Verfahren erzeugte Monolysinat eine hohe Reinheit aufweist. Nach Ausführungsformen der Erfindung ist die gewonnene Monolysinat-Verbindung im Wesentlichen oder vollständig frei von diversen Rückständen, die bei der Verwendung der im Stand der Technik bekannten Herstellungsverfahren in der Regel das Produkt verunreinigen.

Beispielsweise wurde beobachtet, dass das am Markt verfügbare Lysin-Sulfat (Nummer der EU Zulassung als Futtermittelbestandteil: 3.2.5), welches in einem Fermentationsprozess gewonnen wird und welches theoretisch ebenfalls als Ausgangsstoff für die chemische Synthese von Monolysinaten verfügbar ist, einen hohen Grad an Verunreinigungen aufweist, die aus Nebenerzeugnissen der Fermentation herrühren. Diese Verunreinigungen können teilweise an der Bildung einer Verbindung aus Lysin und Metallsalz zu einem Chelat oder einem Salz teilnehmen, wobei die Art und Menge der so gebildeten "Verunreinigungs-Verbindungen" weder vorhergesagt noch verlässlich vermieden werden können. Eine Entfernung dieser undefinierten Verbindungen aus dem Endprodukt ist nur eingeschränkt bis gar nicht möglich.

Demgegenüber wird gemäß Ausführungsformen der Erfindung die flüssige Reaktionsmischung dadurch hergestellt, dass Lysin als reine Substanz und das Metallsalz jeweils in Wasser gelöst werden, wobei das In-Lösung-Bringen des Lysins vor, während oder nach dem In-Lösung-Bringen des Metallsalzes erfolgen kann. Nach bevorzugten Ausführungsformen wird eine wässrige Lysinlösung, die das an der Erzeugung der Verbindung beteilige Metallsalz noch nicht erhält, fertig bereitgestellt und z.B. von kommerziellen Händlern auf dem Markt bezogen. Das Metallsalz wird dann in dieser wässrigen Lysinlösung gelöst, um die flüssige Reaktionsmischung herzustellen.

Die im Stand der Technik bekannten Herstellungsverfahren für Aminosäure-Chelate oder Aminosäure-Salze arbeiten teilweise mit hohen Drücken oder hohen Temperaturen. Lysin ist aber eine recht empfindliche Aminosäure, welche bei diesen extremen Bedingungen oftmals nicht oder nicht zerstörungsfrei verarbeitet werden kann. Ausführungsformen der Erfindung stellen demgegenüber ein vergleichsweise schonendes Herstellungsverfahren dar, welches auch für chemisch empfindliche Aminosäuren wie Lysin geeignet ist.

Nach Ausführungsformen der Erfindung umfasst das Verfahren also eine Bereitstellung einer wässrigen Lysinlösung. Die flüssige Reaktionsmischung wird erzeugt durch Lösen des Metallsalzes in der wässrigen Lysinlösung.

Derzeit am Markt verfügbare wässrige Lysinlösungen ("Flüssig-Lysin") , die durch in-Lösung-Bringen von Lysin in Wasser gewonnen wurden, weisen die mit einer Herstellung von Lysin in Fermentern einhergehenden Verunreinigungen nicht auf, da diese in einem anderen Verfahren hergestellt werden bzw. eventuelle Rückstände im Zuge des Herstellungsprozesses entfernt wurden. Beispielsweise kann es sich bei der Lysinlösung um eine am Markt erhältliche Lösung von reinem oder aufgereinigtem Lysin handeln, welches im Wesentlichen oder völlig frei ist von anderen Substanzen. Die zur Erzeugung der Monolysinat-Verbindung verwendete wässrige Lysinlösung ist also insbesondere eine reine Lösung von Lysin in Wasser, die weitgehend oder vollständig frei ist von anderen Substanzen und Verunreinigungen, und insbesondere frei ist von Fermentationsrückständen. Monolysinat-Verbindungen, die gemäß Ausführungsformen der Erfindung aus den derzeit kommerziell erhältlichen wässrigen Lysinlösungen hergestellt werden, weisen daher einen deutlich höheren Reinheitsgrad auf als entsprechende Chelat-Verbindungen, die aus Lysin-Sulfaten aus einer Fermentation bzw. mittels mechanischer Aktivierung erzeugt wurden.

Abgesehen davon sind handelsübliche Lysin-Sulfat-Verbindungen wesentlich teurer (bezogen auf die Menge an Lysin) als das derzeit am Markt verfügbare "Flüssig-Lysin". Letzteres ist kommerziell als wässrige Lysinlösung mit einem Lysingehalt von etwa 50% Gewichtsanteil (der Rest ist Wasser) am Markt erhältlich, z.B. unter dem Handelsnamen "BestAmino^{™} L-Lysin Liquid Feed Grade" der Firma CJ CHEILJEDANG BIO. Eine wässrige Lysinlösung mit einem Lysin Gehalt von 30-40 % hat bei 20°C einen pH-Wert im Bereich von ca. 9-11.

Lysin ist am Markt außerdem als L-Lysin-Monohydrochlorid, kurz "Lysin-HCl" erhältlich (Nummer der EU Zulassung als Futtermittelbestandteil: 3.2.3). Die Anmelderin hat aber festgestellt, dass Lysin-HCl die zu seiner Verarbeitung verwendeten Maschinen und Geräte, sofern diese aus Metall bestehen oder metallische Komponenten haben, korrodieren lässt. Insbesondere dann, wenn Lysin-HCl Verbindungen thermisch, mechanisch und/oder nasschemisch aktiviert werden, ist ein starker korrodierender Effekt zu beobachten. Dieser bewirkt nicht nur einen erhöhten Materialverschleiß, Korrosionsschäden an Behältnissen und Maschinen und damit erhöhte Herstellungskosten, sondern auch einen Eintrag von Rost und anderen korrosionsbedingten Verunreinigungen, insb. Schwermetallen, in das Reaktionsprodukt. Somit ermöglicht das erfindungsgemäße Herstellungsverfahren die Herstellung von besonders reinen Monolysinat-Verbindungen, und dies mit geringeren Herstellungskosten bzw. geringerem Materialverschleiß aufgrund von Korrosion.

In einem weiteren vorteilhaften Aspekt haben wässrige Lysinlösungen einen basischen pH-Wert, sodass Korrosionsschäden an den Maschinen und Geräten während des Herstellungsverfahrens der Monolysinat-Verbindung vermieden werden.

Nach Ausführungsformen der Erfindung wird die flüssige Reaktionsmischung in einem metallischen Behältnis erzeugt, in welchem auch die Umsetzung der Edukte der Reaktionsmischung zur finalen Monolysinat-Verbindung erfolgt. Alternativ dazu können auch mehrere metallische Behältnisse zur Erzeugung der Reaktionsmischung und zur Durchführung der chemischen Umsetzung verwendet werden und/oder es können metallische Gerätschaften, zum Beispiel Rührer, Mixer oder dergleichen zum Einsatz kommen. Dies kann vorteilhaft sein, weil eine Vielzahl von am Markt erhältlichen Reaktoren und Behältnissen zur Durchführung und Kontrolle chemischer Reaktionen aus Metall sind. Diese Standardbehältnisse können verwendet werden, ohne dass mit einem vorzeitigen Ausfall dieser Behältnisse oder Gerätschaften gerechnet werden muss, da die wässrige Lysinlösung aufgrund ihres alkalischen pH-Wertes und/oder aufgrund der Abwesenheit von Chloriden (Salzen der Salzsäure) nicht zu einer Korrosion dieser metallischen Gegenstände führt.

Nach Ausführungsformen der Erfindung handelt es sich bei dem hier beschriebenen Lysin insbesondere um L-Lysin (CAS-Nr. 56-87-1), welches in Wasser gelöst vorliegt. Lysin hat die Summenformel C₆H₁₄N₂O₂ und ein Molekulargewicht von 146,19 g/Mol.

Bei dem Metallsalz handelt es sich um ein Metallsulfat, ein Metallhydroxid oder ein Metallcarbonat.

Die genannten Metallsalze haben gegenüber anderen Salzen, insbesondere Metallchloriden, den Vorteil, dass diese keine oder nur eine geringfügig erhöhte Korrosionsrate von Metallgegenständen bewirken, welche mit der flüssigen Reaktionsmischung in Kontakt kommen.

Bei dem Metall des Metallsalzes handelt es sich um eines der in Anspruch 1 genannten zweiwertigen Metalle. Insbesondere kann es sich bei dem Metallsalz um ein Metallsulfat handeln.

Nach Ausführungsformen der Erfindung handelt es sich bei der Monolysinat-Verbindung um ein Monolysinat-Salz. Jede monomere Einheit des Salzgitters hat also genau ein Lysinmolekül und vorzugsweise genau ein Metallatom (des Metallsalzes).

Nach Ausführungsformen der Erfindung handelt es sich bei der Monolysinat-Verbindung um ein monomeres Monolysinat-Salz, oder um ein polymeres Monolysinat-Salz, oder um eine Mischung aus monomerem und polymerem Monolysinat-Salz.

Nach Ausführungsformen der Erfindung handelt es sich bei der Monolysinat-Verbindung um ein Mangan-Monolysinatsulfat oder um ein Eisen-Monolysinatsulfat.

Nach besonders bevorzugten Ausführungsformen der Erfindung handelt es sich bei dem Metallsalz um ein Zinksulfat (ZnSO₄), Eisensulfat (FeSO₄) oder Mangansulfat (MnSO₄). Es kann sich aber auch um ein Kalziumsulfat (CaSO₄), Magnesiumsulfat (MnSO₄), Kobaltsulfat (CoSO₄), Natriumsulfat (NaSO₄), oder Nickelsulfat (NiSO₄) handeln.

Die flüssige Reaktionsmischung wird durch Lösen eines Metallsalzes in der wässrigen Lysinlösung in einem molaren Verhältnis von 1 Mol eines Metallatoms des Metallsalzes zu 1 Mol des Lysins erzeugt.

Die jeweiligen Gewichtsverhältnisse von Metallsalz und Lysin in der Reaktionsmischung hängen von dem jeweils verwendeten Metall bzw. Metallsalz ab. In Tests wurde beobachtet, dass eine flüssige Reaktionsmischung, die ein molares Verhältnis von Lysin:Metallatom aufwies, das von dem oben genannten 1:1 Verhältnis deutlich abwich, dennoch im Wesentlichen strukturreine Produkte gemäß der Formeln in Bildern 2, 4, 6 oder 8 bildeten, auch wenn ggf. in der Reaktionsmischung in diesem Fall dann Rückstände nichtreagierter Metallsalzkomponenten verblieben. Das erfindungsgemäße Verfahren gemäß Ausführungsformen der Erfindung kann also vorteilhaft sein, da es robust gegenüber Schwankungen des molaren Verhältnisses von Lysin und Metall bzw. Metallsalz ist. Vorzugsweise wird jedoch die flüssige Reaktionsmischung so erzeugt, dass Lysin und Metallatom in einem molaren Verhältnis von 1:1 vorliegen, um zu gewährleisten, dass möglichst sämtliche Edukte, zumindest sämtliche Metallatome des Metallsalzes, in der Reaktion zu dem gewünschten Produkt, der besagten Monolysinat-Verbindung, umgesetzt werden.

Nach Ausführungsformen der Erfindung umfasst der Prozess der chemischen Umsetzung eine mechanische Durchmischung des gelösten Lysins und des gelösten Metallsalzes bei einer Temperatur von mindestens 60°C, vorzugsweise 60°C - 90°C für mindestens 15 Minuten. Beispielsweise kann die flüssige Reaktionsmischung mindestens 20 Minuten lang, insbesondere etwa 25-35 Minuten lang, bei einer Temperatur von etwa 80°C gerührt werden. Es wurde beobachtet, dass eine Rührdauer von weniger als 60 Minuten, und auch von weniger als 50 Minuten, und auch von weniger als 40 Minuten in der Regel ausreichend ist, um alle Edukte in der flüssigen Reaktionsmischung vollständig oder nahezu vollständig in die Monolysinat-Verbindung umzusetzen.

Nach Ausführungsformen der Erfindung weist die bereitgestellte wässrige Lysinlösung einen Lysinanteil von mindestens 30 Gewichtsprozent, vorzugsweise mindestens 40% Gewichtsprozent der wässrigen Lösung auf. Der Rest der wässrigen Lysinlösung besteht im Wesentlichen oder vollständig aus Wasser. Beispielsweise kann eine wässrige Lösung mit einem 50%igen Lysinanteil kommerziell im Handel bezogen werden. Diese wässrige Lösung kann dann durch Zugabe von Wasser weiter verdünnt werden, um das Versprühen der vollständig umgesetzten flüssigen Reaktionsmischung in einem späteren Sprühtrocknungsverfahren und/oder das Lösen des Metallsalzes in der wässrigen Lysinlösung zu erleichtern. Das zusätzliche Wasser, sofern dies überhaupt erforderlich sein sollte, wird vorzugsweise der bereitgestellten wässrigen Lysinlösung zugesetzt, bevor das Metallsalz in der wässrigen Lysinlösung aufgelöst wird. Es ist aber auch möglich, dass zusätzliche Wasser zusammen mit oder erst nach dem Metallsalz zuzugeben.

Die Frage, ob und ggf. wie viel Wasser zugegeben werden muss, hängt von dem gewählten Trocknungsverfahren, der Konzentration der bezogenen flüssig-Lysinlösung, sowie der Art des Metallsalzes, welches darin gelöst werden soll, ab. Zur Beantwortung dieser Frage sind aber einfache experimentelle Handgriffe völlig ausreichend. Falls sich die berechnete Menge an Metallsalz beispielsweise auch nach 15 Minuten Rühren bei Temperaturen über 60° nicht vollständig in der wässrigen Lysinlösung gelöst haben sollte, kann immer wieder eine kleine Menge Wasser zugegeben werden, bis sich das Metallsalz vollständig gelöst hat. Die Menge des zugegebenen Wassers wird notiert, und kann bei nachfolgenden Herstellungsprozessen mit dem gleichen Typ von wässriger Lysinlösung und dem gleichen Typ von Metallsalz von Anfang an abgemessen und der wässrigen Lysinlösung oder der Reaktionsmischung zugegeben werden. Wird zur Trocknung der vollständig umgesetzten Reaktionsmischung ein Vernebelungsprozess verwendet und wird dabei festgestellt, dass die Reaktionsmischung zu dickflüssig für die verwendeten Düsen ist, sodass die Nebelbildung unterbleibt oder unzureichend ausfällt, kann bei nachfolgenden Herstellungsprozessen mit dem gleichen Typ von wässriger Lysinlösung und dem gleichen Typ von Metallsalz der wässrigen Lysinlösung oder der Reaktionsmischung zusätzliches Wasser zugegeben werden. Läuft der Vernebelungsprozess nun wie gewünscht, wird die zusätzliche Menge an Wasser notiert und in der Zukunft bei einer Wiederholung des Herstellungsprozesses immer der Lysinlösung oder der Reaktionsmischung zugegeben. Funktioniert der Vernebelungsprozess wegen zu hoher Viskosität der Reaktionsmischung nach wie vor nicht richtig, wird die zusätzliche Menge an Wasser bei der nächsten Durchführung des Verfahrens weiter erhöht. Die chemische Reaktion samt anschließender Vernebelung wird so oft wiederholt und die Wassermenge ggf. angepasst, bis der gewünschte Vernebelungseffekt erzielt wird.

In manchen Ausführungsformen wird bereits die bereitgestellte wässrige Lysinlösung auf über 60° erhitzt bevor das Metallsalz darin aufgelöst wird, da dies den Lösungsprozess des Metallsalzes beschleunigen kann. Es ist aber auch möglich, zuerst das Metallsalz in die wässrige Lysinlösung zu überführen um das Metallsalz darin aufzulösen und nach oder während dieses Lösungsprozesses die Temperatur dieser Lösung auf über 60° zu erhöhen. Vorzugsweise wird das Metallsalz unter ständigem Rühren in der flüssigen Lysinlösung gelöst.

Nach Ausführungsformen der Erfindung sind die wässrige Lysinlösung und die flüssige Reaktionsmischung im Wesentlichen frei sind von Salzsäure und deren Salzen. Insbesondere sind die wässrige Lysinlösung und die flüssige Reaktionsmischung im Wesentlichen frei von Lysin-HCL Salzen bzw. deren Lösungsprodukten.

Dies kann vorteilhaft sein, da insbesondere Cl⁻ Ionen, Salzsäure und deren Salze bzw. Lysin-HCL Rückstände eine Korrosion von Metallgegenständen wie zum Beispiel metallischen Reaktionsgefäßen oder Rührern bewirken können.

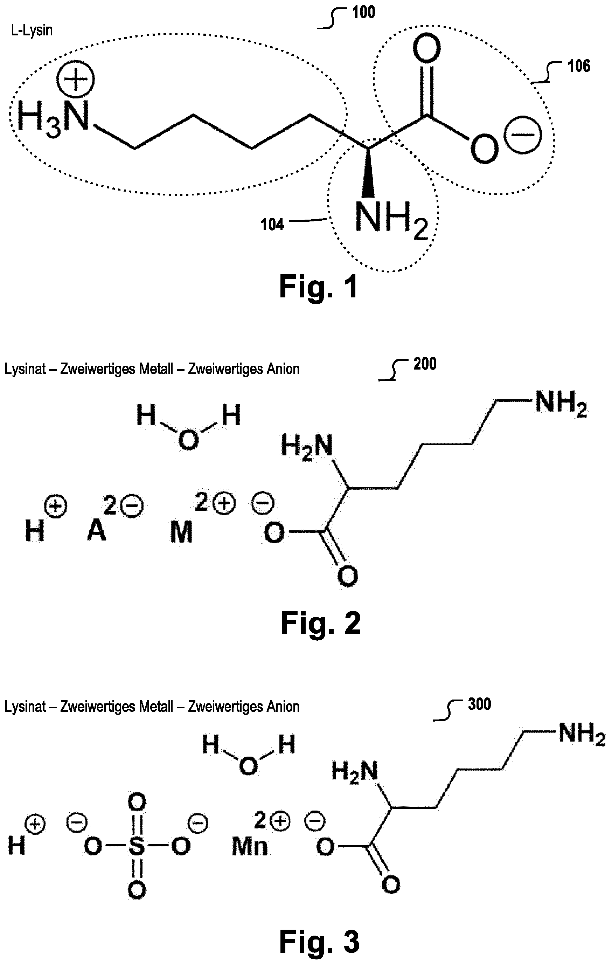

Nach Ausführungsformen der Erfindung ist die nach der Umsetzung in der flüssigen Reaktionsmischung enthaltene Monolysinat-Verbindung eine Verbindung gemäß einer Strukturformel, wie sie in einer der Figuren 2-9 allgemein bzw. als konkretes Beispiel dargestellt ist. Typischerweise enthält die flüssige Reaktionsmischung weniger als 1% Monolysinat-Verbindungen mit einer abweichenden Strukturformel oder einem abweichenden molaren Verhältnis von Lysin und Metallatom.

Nach Ausführungsformen der Erfindung umfasst das Verfahren ferner eine Erzeugung eines Granulats aus der Monolysinat-Verbindung nach oder während der Trocknung.

In einem weiteren Aspekt betrifft die Erfindung eine Monolysinat-Verbindung, die durch ein Verfahren zur Erzeugung einer Monolysinat-Verbindung gemäß Patentanspruch 1 und diesbezügliche Ausführungsformen erzeugt wurde.

Nach Ausführungsformen der Erfindung hat die Monolysinat-Verbindung eine Strukturformel wie in einer der Figuren 2, 4, 6 und 8 dargestellt, wobei M für das metallische Kation des Metallsalzes und A für das Anion des Metallsalzes steht. Konkrete Beispiele hierfür sind jeweils in den Figuren 3, 5, 7 und 9 dargestellt.

Nach Ausführungsformen der Erfindung ist die Monolysinat-Verbindung im Wesentlichen oder vollständig frei von Chloriden und Cl⁻ - Ionen. Vorzugsweise sind die vollkommen ausreagierte flüssige Reaktionsmischung und die aus dieser gewonnenen getrockneten Reaktionsprodukte im Wesentlichen oder vollständig frei von Chloriden und Chlor-Ionen. Insbesondere ist das im Trockungs- und/oder Granulationsprozess erhaltene Pulver bzw. Granulat im Wesentlichen oder vollständig frei von Chloriden und Chlor-Ionen.

Die Monolysinat-Verbindung, die mit dem hier beschriebenen Verfahren gemäß Patentanspruch 1 erzeugt wird, enthält Kristallwasser. Die Menge des Kristallwassers liegt typischerweise im Bereich von 5 bis maximal 10 % des Gewichts der Monolysinat-Verbindung. Es handelt sich bei der Monolysinat-Verbindung also um ein Hydrat. Dies kann vorteilhaft sein, da Hydrate wenig bis gar nicht hygroskopisch sind. In getrockneter Form neigt die so hergestellte Monolysinat-Verbindung also nicht dazu, Feuchtigkeit aus der Umgebungsluft aufzunehmen und Klumpen zu bilden. Die Verbindung bleibt also rieselfähig und damit über lange Zeit hinweg gut lagerbar und verarbeitungsfähig.

In einem weiteren Aspekt betrifft die Erfindung eine Substanzmischung, die eine oder mehrere Monolysinat-Verbindungen gemäß Patentanspruch 8 beinhaltet. Bei der Substanzmischung kann es sich z.B. um ein Futter oder einen Futtermittelzusatz für Nutz- und Haustiere, einen Gärzusatz für Kompostier- oder Biogasanlagen, einen Pflanzendünger, ein Nahrungsmittel, einen Nahrungsmittelzusatz oder um ein Nahrungsergänzungsmittel für Menschen handeln.

In einem weiteren Aspekt betrifft die Erfindung eine Verwendung einer Monolysinat-Verbindung gemäß Patentanspruch 8 als Futtermittelzusatz für Nutz- und Haustiere und/oder als Gärzusatz und/oder als Düngemittelzusatz und/oder als Nahrungsmittelzusatz und/oder als Nahrungsergänzungsmittel.

Die Verwendung der Monolysinat-Verbindung in der Tierernährung kann eine Leistungssteigerung und eine verbesserte intestinale Absorption von Spurenelementen bewirken. Die Effizienz von Spurenelementen im Futter lässt sich verbessern und die Ausscheidungsrate reduzieren. Das Risiko einer physiologischen Unterversorgung und Leistungsdepression werden verringert. Darüber hinaus scheinen organisch gebundene Spurenelemente physiologische Vorteile zu haben, z. B. verbesserte zootechnische und reproduktive Leistungen, höhere Ei-Qualität, sowie höhere Inkorporation der Spurenelemente in Körperorgane oder Gewebe.

Eine "ionische Bindung" (auch "Ionenbindung", "Salzbindung", "heteropolare Bindung" oder "elektrovalente Bindung") ist eine chemische Bindung, die aus der elektrostatischen Anziehung positiv und negativ geladener Ionen resultiert. Ab einer Elektronegativitäts-Differenz von ΔEN=1,7 spricht man von einem 50% partiell ionischen Charakter. Bei einer Differenz größer als 1,7 liegen demnach ionische Bindungen, darunter polare, überwiegend kovalente Bindungen vor. Der Fall der reinen ionischen Bindung stellt jedoch eine Idealisierung dar. Es kommt - typischerweise - eine Ionenbindung zwischen Elementen, die links im Periodensystem (PSE) stehen, also Metallen, und Elementen, die rechts im PSE stehen, Nichtmetallen, zustande. Schaut man sich den Ionenbindungsanteil z. B. von Natriumchlorid an, welches oft als klassischer Fall der Ionenbindung angesehen wird, so errechnet sich ein Wert von ca. 73 %. Der ionische Charakter von Cäsiumfluorid liegt bei ca. 92 %.

Spektroskopische Aufnahmen haben gezeigt, dass es sich bei der Monolysinat-Verbindung gemäß Ausführungsformen der Erfindung um ein auf ionischen Bindungen beruhendes Monolysinat handelt, nicht um eine Komplexverbindung mit koordinativen Bindungen oder um ein Chelat im engeren Sinne.

Eine "Monolysinat-Verbindung" ist eine Verbindung aus einem oder mehreren Monomeren, wobei in jedem Monomer genau ein Lysinmolekül über ionische Bindungen mit genau einem Metallsalz bzw. dessen ionischen Komponenten verbunden ist.

Eine "Chelat-Verbindungen", auch als "Chelatkomplex" oder "Chelat" bezeichnet, ist eine Verbindung, bei der ein mehrzähniger Ligand (auch "Chelator", besitzt mehr als ein freies Elektronenpaar) mindestens zwei Koordinationsstellen (Bindungsstellen) des Zentralatoms einnimmt. Beim Zentralatom handelt es sich vorzugsweise um ein zweifach positiv geladenes Metallion (etwa Fe2+, Zn2+). Liganden und Zentralatom sind über koordinative Bindungen verknüpft. Das bedeutet, das bindende Elektronenpaar wird allein vom Liganden bereitgestellt. Chelatkomplexe sind stabiler als gleiche Komplexe mit einzähnigen, nicht untereinander verknüpften Liganden. Die koordinative Bindung (Komplexbindung) zwischen Ligand und Metall kann als polare kovalente Bindung betrachtet werden und unterscheidet sich von anderen Formen der chemischen Bindung: anders als im Falle der koordinativen Bindung weist eine Ionenbindung gar kein bindendes Elektronenpaar auf. Anders als im Falle der koordinativen Bindung steuert in einer unpolaren kovalenten Bindung jeder Bindungspartner ein Elektron zum bindenden Elektronenpaar bei.

Unter einem Stoff, der "im Wesentlichen frei ist" von einer bestimmten Substanz, wird im Folgenden ein Stoff verstanden, der zu weniger als 1 %, vorzugsweise zu weniger als 0.4 % seines Gewichts aus dieser Substanz besteht bzw. diese Substanz zu weniger als 1 %, vorzugsweise zu weniger als 0.4 % seines Gewichts beinhaltet.

Unter einer "wässrigen Lysinlösung" wird hier Wasser verstanden, in welchem Lysin gelöst ist. Nach bevorzugten Ausführungsformen ist die wässrige Lysinlösung völlig oder im Wesentlichen frei von anderen Aminosäuren außer Lysin. Nach Ausführungsformen der Erfindung ist die flüssige Reaktionsmischung völlig oder im Wesentlichen frei von Chloriden und Cl⁻ - Ionen.

Unter einer "flüssigen Reaktionsmischung" wird hier eine wässrige Lösung verstanden, welche die Edukte und/oder Produkte dieser chemischen Reaktion beinhaltet. Vor dem Start der chemischen Reaktion beinhaltet die Reaktionsmischung im Wesentlichen nur die Edukte. Nach vollständiger Umsetzung aller oder zumindest der limitierenden Edukte zu einem oder mehreren Produkten beinhaltet die Reaktionsmischung die Produkte und ggf. als stöchiometrischer Rest verbliebene Edukte.

### Kurzbeschreibung der Figuren

Im Folgenden werden Ausführungsformen der Erfindung nur exemplarisch näher erläutert, wobei auf die Zeichnungen verwiesen wird, in denen sie enthalten sind. Es zeigen:
- Figur 1: eine allgemeine Strukturformel von L-Lysin;
- Figur 2: eine allgemeine Strukturformel einer Monolysinat-Verbindung;
- Figur 3: ein konkretes Beispiel einer Monolysinat-Verbindung gemäß der allgemeinen Formel von Figur 2;
- Figur 4: eine allgemeine Strukturformel einer weiteren Monolysinat-Verbindung;
- Figur 5: ein konkretes Beispiel einer Monolysinat-Verbindung gemäß der allgemeinen Formel von Figur 4;
- Figur 6: eine allgemeine Strukturformel einer weiteren Monolysinat-Verbindung;
- Figur 7: ein konkretes Beispiel einer Monolysinat-Verbindung gemäß der allgemeinen Formel von Figur 6;
- Figur 8: eine allgemeine Strukturformel einer weiteren Monolysinat-Verbindung;
- Figur 9: ein konkretes Beispiel einer Monolysinat-Verbindung gemäß der allgemeinen Formel von Figur 8;
- Figur 10: ein Flussdiagramm eines Verfahrens zur Herstellung einer MonolysinatVerbindung wie z.B. in Figur 1 dargestellt;
- Figur 11: ein Flussdiagramm eines verallgemeinerten Verfahrens zur Herstellung einer Monolysinat-Verbindung;
- Figur 12: ein Behältnis mit einer flüssigen Reaktionsmischung;
- Figur 13: Fotos von Mangan-Monolysinat-Granulat;
- Figur 14: Fotos von Eisen-Monolysinat-Granulat;
- Figur 15: elektronenmikroskopische Aufnahmen von Eisen-Monolysinat-Sulfat;
- Figur 16: elektronenmikroskopische Aufnahmen von Mangan-Monolysinat-Sulfat;
- Figur 17: ein IR Spektrum von zwei Monolysinaten.

### Ausführliche Beschreibung

Figur 1 zeigt die essentielle proteinogene α-Aminosäure Lysin in seiner natürlichen L-Form. Gemäß bevorzugten Ausführungsformen der Erfindung ist das "Lysin" als L-Lysin zu verstehen. Lysin beinhaltet die für Aminosäuren charakteristischen Gruppen, nämlich eine Aminogruppe 104, eine Carboxylgruppe 106 sowie den für Lysin typischen Rest 102. Die Carboxylgruppe kann negativ geladen sein, sodass Lysin als Lysin-Anion vorliegt.

**Figur 2** stellt eine allgemeine Strukturformel einer Monolysinat-Verbindung 200 dar, wie sie nach Ausführungsformen des hier beschriebenen Herstellungsverfahrens gewonnen werden kann.

Die Monolysinat-Verbindung 200 beinhaltet genau ein Lysinmolekül pro Metallatom. Sie entsteht beispielsweise aus einer wässrigen Lösung von Lysin und einem Metallsalz, wobei das Metallsalz aus einem zweiwertigen Metall und einem zweiwertigen Anion besteht.

Die Carboxylgruppe ist negativ geladen, sodass Lysin als Lysin-Anion vorliegt. Das Lysin-Anion ist mit dem Metallkation des gelösten Metallsalzes über eine ionische Bindungen verbunden. Insbesondere stellt in der ionischen Verbindung das metallische Kation M das Bindeglied zwischen dem Anion A des Metallsalzes und dem Lysinanion dar. In der Ausführungsform nach Figur 2 ist das Metallion M ein zweifach positiv geladenes Kation und das Metallsalzantion ein zweifach negativ geladenes Anion, dessen eine Ladung durch ein Proton mit einer Ladung abgesättigt ist. Bei dem positiv geladenen Metallion kann es sich insbesondere um Mn²⁺, Fe²⁺, Zn²⁺, Ca²⁺, Mg²⁺, Co²⁺, Na²⁺, oder Ni²⁺ handeln. Das Anion kann zum Beispiel aus einem Sulfatrest oder Carbonatrest bestehen.

**Figur 3** zeigt ein konkretes Beispiel einer Monolysinat-Verbindung gemäß der allgemeinen Formel von Figur 2, nämlich eine Strukturformel für Mangan-Monolysinat-Sulfat 300. Die Monolysatverbindung 200, 300 kann Kristallwasser (Hydratwasser) beinhalten.

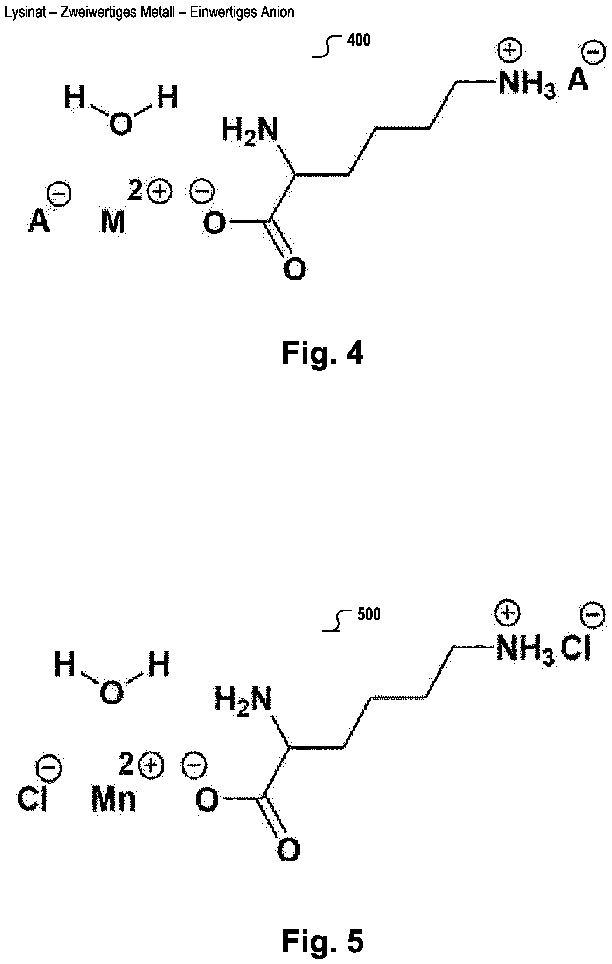

**Figur 4** stellt eine allgemeine Strukturformel einer Monolysinat-Verbindung 400 dar, wie sie nach Ausführungsformen des hier beschriebenen Herstellungsverfahrens gewonnen werden kann. Die Monolysinat-Verbindung 400 beinhaltet genau ein Lysinmolekül pro Metallatom. Sie entsteht beispielsweise aus einer wässrigen Lösung von Lysin und einem Metallsalz, wobei das Metallsalz aus einem zweiwertigen Metall und zwei einwertigen Anionen besteht.

**Figur 5** zeigt ein konkretes Beispiel einer Monolysinat-Verbindung gemäß der allgemeinen Formel von Figur 4, nämlich eine Strukturformel für Mangan-Monolysinat-Chlorid 500. Die Monolysatverbindung kann Kristallwasser (Hydratwasser) beinhalten. Die Verbindung 500 kann als Reaktionsprodukt aus Flüssiglysin und Manganchlorid MnCl₂ gewonnen werden. Eines der beiden Chloridionen des MnCl₂ Salzes lagert sich an das Amin im Aminosäurerest des Lysins an, es entsteht eine ionische Bindung zwischen der NH3+ Gruppe und dem Chlorid-Anion. Das andere Chloridion lagert sich an das zweiwertige Mangan-Kation an, es entsteht eine ionische Bindung zwischen dem Metallkation und dem Chlorid-Anion. Da die Anionen und Kationen des MnCl₂ Salzes sich in Lösung befinden, bevor das Salz gebildet wird, können die beiden Chloridionen auch von unterschiedlichen MnCl₂ Salzmonomeren stammen. Aufgrund der korrosionsfördernden Eigenschaft von Chloriden ist diese Ausführungsform mit Chloriden als Anion möglich, jedoch nicht bevorzugt.

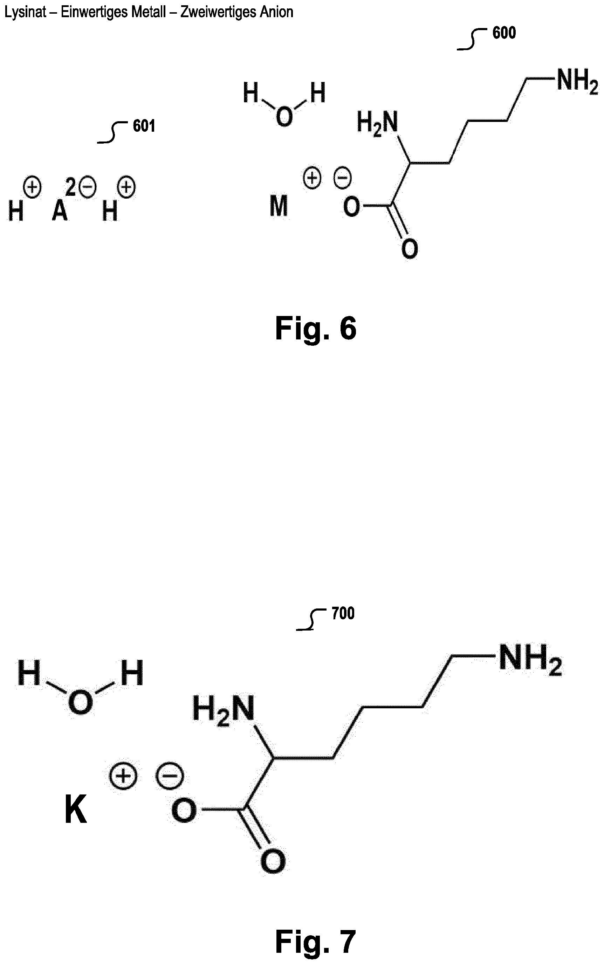

**Figur 6** zeigt eine allgemeine Strukturformel einer Monolysinat-Verbindung 600, wie sie nach Ausführungsformen des hier beschriebenen Herstellungsverfahrens gewonnen werden kann. Die Monolysinat-Verbindung 600 beinhaltet genau ein Lysinmolekül pro Metallatom. Sie entsteht beispielsweise aus einer wässrigen Lösung von Lysin und einem Metallsalz, wobei das Metallsalz aus zwei einwertigen Metallatomen und einem zweiwertigen Anion (z.B. Sulfat, Carbonat) besteht. Die Verbindung 600, 700 kann Kristallwasser enthalten. Das Lysinmolekül ist über seine einfach negativ geladene Carboxylgruppe mit dem einfach positiv geladenen Metallion über eine ionische Bindung verbunden. Das zweiwertige Anion ist nicht Bestandteil der Monolysinat-Verbindung 600, sondern verbleibt als Rückstand 601 in der Reaktionslösung.

Die Strukturformel 600 gibt die Struktur wieder, wie sie beispielsweise gemäß Ausführungsformen des erfindungsgemäßen Verfahrens entsteht, wenn als Metallsalz Kaliumsulfat (K₂SO₄) verwendet wird.

**Figur 7** zeigt ein konkretes Beispiel einer Monolysinat-Verbindung gemäß der allgemeinen Formel 600 von Figur 6, nämlich eine Strukturformel für Kalium-Monolysinat 700. Die Monolysatverbindung 700 kann Kristallwasser (Hydratwasser) beinhalten.

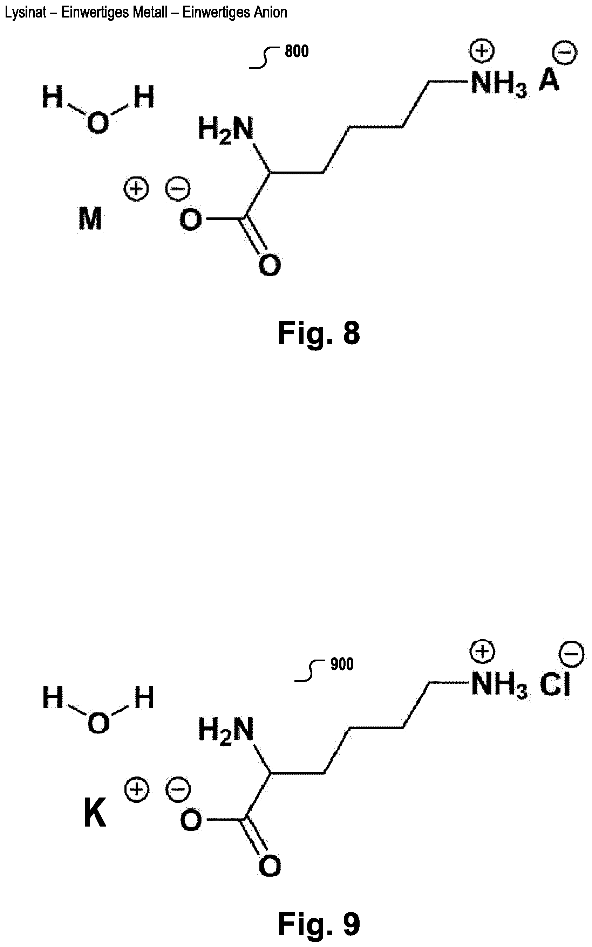

**Figur 8** zeigt eine allgemeine Strukturformel einer Monolysinat-Verbindung 800, wie sie nach Ausführungsformen des hier beschriebenen Herstellungsverfahrens gewonnen werden kann. Die Monolysinat-Verbindung 800 beinhaltet genau ein Lysinmolekül pro Metallatom. Sie entsteht aus einer wässrigen Lösung von Lysin und einem Metallsalz, wobei das Metallsalz beispielsweise aus einem einwertigen Metallatom und einem einwertigen Anion (z.B. Chloridion) besteht. Die Verbindung 800, 900 kann Kristallwasser enthalten. Das Lysinmolekül ist über seine einfach negativ geladene Carboxylgruppe mit dem einfach positiv geladenen Metallion über eine ionische Bindung verbunden.

**Figur 9** zeigt ein konkretes Beispiel einer Monolysinat-Verbindung gemäß der allgemeinen Formel 800 von Figur 8, nämlich eine Strukturformel für Kalium-Chlorid-Monolysinat 900. Die Monolysatverbindung 900 kann Kristallwasser (Hydratwasser) beinhalten.

Das freie Elektronenpaar am Stickstoffatom der Amingruppe des Lysinrests kann ein Proton aufnehmen wie beispielhaft für Figur 8 gezeigt.

Die Monolysinat-Verbindung gemäß Ausführungsformen der Verbindung kann als Monomer vorliegen oder als Polymer, das mehrere der besagten Monomere umfasst. Die Verbindung kann auch als eine Mischung aus Monomer und Polymer vorliegen. Beispielsweise können die Monomere aus einer Verbindung gemäß der in einer der Figuren 2-9 spezifizierten Formel ausgebildet sein. Die Ausbildung von polymeren Lysinatsalzen kann vorteilhaft sein, da auf diese Weise ein homogenes Salzkristallgitter und eine dichte Packung der Lysinatsalzmonomere erzielt werden kann.

In manchen Ausführungsformen kann das Metallsalz, das der wässrigen Lysinlösung zugegeben wird, auch Hydratwasser beinhalten.

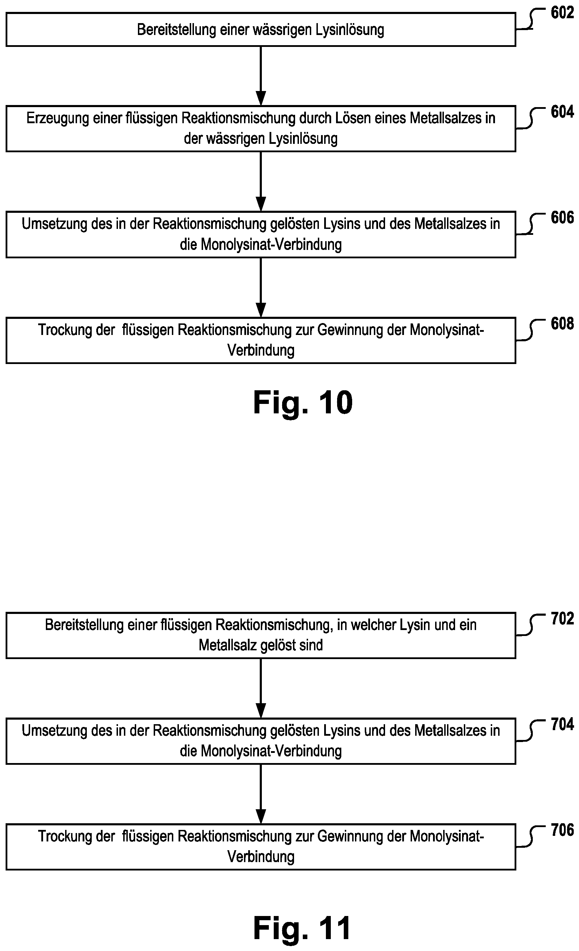

**Figur 10** ist ein Flussdiagramm eines Verfahrens zur Herstellung einer Monolysinat-Verbindung 200, 300, 400, 500, 600, 700, 800, 900 wie z.B. in Figuren 2-9 dargestellt.

In einem ersten Schritt 602 wird eine wässrige Lysinlösung bereitgestellt. Beispielsweise kann die wässrige Lysinlösung über kommerzielle Anbieter bezogen werden. Auf dem Markt sind beispielsweise wässrige Lysinlösungen mit einem Lysingehalt von ca. 50 Gewichtsprozent erhältlich. Die wässrige Lysinlösung hat eine dunkelbraune Färbung und einen alkalischen bis leicht alkalischen pH-Wert. Lösungen mit einem solchen pH-Wert greifen Stahlbehältnisse nicht bzw. kaum an. Wässrige Lysinlösungen sind über einen langen Zeitraum hinweg stabil gelöst und lagerfähig, zum Beispiel in Plastik- oder Stahltanks. Nach manchen Ausführungsformen ist es möglich, die wässrige Lysinlösung selbst zu erstellen, indem die gewünschte Menge Lysin in Wasser oder einer wässrigen Metallsalzlösung gelöst wird. Die Verwendung einer bereits vorgefertigten wässrigen Lysinlösung (ohne Metallsalz) bewirkt jedoch eine Zeitersparnis, da derartige Lösungen bereits fertig am Markt bezogen werden können.

Insbesondere soll die verwendete wässrige Lysinlösung frei sein von Chloriden und Cl⁻ - Ionen und sonstigen Substanzen, die eine Korrosion von Stahlbehältnissen oder Geräten bewirken könnten. Hierdurch wird eine Korrosion der Anlagen verhindert, welche nicht nur im Hinblick auf die Lebensdauer der Anlagen unerwünscht ist, sondern auch im Hinblick auf die Qualität der zu gewinnenden Monolysinat-Verbindung: bei im Stand der Technik verwendeten nasschemischen Verfahren zur Chelat-Bildung, bei welchen Salzsäure oder die Salze derselben in der Reaktionsmischung enthalten sind, wurde beobachtet, dass die durch die Salzsäure bewirkte Korrosion der Anlagen zu einer Freisetzung von Stahlveredlern wie Chrom und anderen Schwermetallen führt. Die durch Korrosion freigesetzten Schwermetalle können ihrerseits auf undefinierte Weise mit den in der Reaktionslösung gelösten Metallsalzen reagieren und Verbindungen bilden, die als Verunreinigungen im Endprodukt enthalten sind. Trockne Verfahren verwenden Mühlen oder andere Formen der mechanischen Aktivierung und führen zu Verunreinigung durch Abrieb. Gerade im Kontext der Verwendung der Monolysinat-Verbindungen als Futtermittelzusatz, Nahrungsergänzungsmittel und/oder als Pflanzendünger ist die Einbringung von Schwermetallen wie Chrom in das Endprodukt in hohem Grade unerwünscht. Viele Schwermetalle sind gesundheitsschädlich und sollten also nicht in den Stoffwechsel von Tier und Mensch bzw. in Ackerböden eingebracht werden. Die Verwendung von einer wässrigen Lysinlösung zur Herstellung von Monolysinaten ist also nicht nur kostengünstig, sondern auch besonders gesund und umweltfreundlich, da Korrosionsprozesse und der damit verbundene Eintrag von unerwünschten Schwermetallen in die Reaktionslösung vermieden wird.

Vorzugsweise handelt es sich bei der bereitgestellten flüssigen Lysinlösung um eine futtermittelrechtlich und/oder lebensmittelrechtlich zugelassene Lysinlösung.

In einem optionalen Schritt kann der bereitgestellten wässrigen Lysinlösung weiteres Wasser zugegeben werden, um die Lösung zu verdünnen. Insbesondere wenn die Lysinlösung im Handel bezogen wird, kann es sein, dass die Konzentration reduziert werden muss, um die Löslichkeit der Metallsalze zu erhöhen und/oder um eine gute Vernebelbarkeit der Reaktionslösung in einem anschließenden Trocknungsprozess sicherzustellen. Es ist auch möglich, dieses zusätzliche Wasser, sofern nötig, vor, während oder nach dem Lösen der Metallsalze in die wässrige Lysinlösung zuzugeben.

In einem weiteren Schritt 604 folgt die Erzeugung einer flüssigen Reaktionsmischung durch Lösen eines Metallsalzes in der wässrigen Lysinlösung. Beispielsweise kann das Salz unter ständigem Rühren der Lysinlösung zugegeben werden. Vorzugsweise hat die Temperatur der wässrigen Lysinlösung während des Löseprozesses des Metallsalzes eine Temperatur von über 30 °C, vorzugsweise über 50 °C, weiterhin vorzugsweise über 60 °C. Vorzugsweise wird das Metallsalz in einem molaren Verhältnis von Metallatom M:Lysin von 1:1 zugegeben. Die Menge des benötigten Metallsalzes hängt dabei von der Art des verwendeten Metallsalzes und der Konzentration der Lysinlösung ab.

Beispielsweise kann die flüssige Reaktionsmischung folgende Komponenten umfassen oder aus diesen bestehen, um ca. 1 kg Mangan-Monolysinat zu erhalten:
- 570 g Mangansulfat Monohydrat (57%)
- 980 g wässrige Lysinlösung mit einem Lysin-Gewichtsanteil von 50 %
- 700 g zusätzliches Wasser.

Gemäß einem weiteren Beispiel kann die flüssige Reaktionsmischung folgende Komponenten umfassen oder aus diesen bestehen, um ca. 1 kg Eisen-Monolysinat zu erhalten:
- 570 g Eisensulfat Monohydrat (57%)
- 980 g wässrige Lysinlösung mit einem Lysin-Gewichtsanteil von 50 %
- 700 g zusätzliches Wasser.

In einem nächsten Schritt 306 erfolgt eine chemische Umsetzung des in der Reaktionsmischung gelösten Lysins und des Metallsalzes, sodass die Monolysinat-Verbindung erzeugt wird. Das gelöste Lysin wird dabei mit dem Metallsalz unter ständigem Rühren bei Temperaturen von vorzugsweise über 60 °C zur Reaktion gebracht und in eine ionische Monolysinat-Metallsalsverbindung überführt. Der Rührvorgang wird vorzugsweise so lange fortgeführt, bis die Edukte der Reaktionsmischung vollständig in die Monolysinat-Verbindung überführt wurden oder bis zur Erreichung des chemischen Gleichgewichts, sodass mit einer Erhöhung der Konzentration der Monolysinat-Verbindung nicht mehr zu rechnen ist. Typischerweise ist hierfür ein Zeitraum von 20-60 Minuten, insbesondere von ca. 25-35 Minuten, erforderlich. Alternativ zu dem Rühren können auch andere Formen der mechanischen Durchmischung der flüssigen Reaktionsmischung verwendet werden, zum Beispiel Schütteln, Verwirbelungen mittels Düsen, wiederholtes Überführen der flüssigen Mischung in andere Behältnisse o. ä.

Beispielhaft ist die chemische Umsetzung von Lysin und Metallsalz zu einer Mono-lysinat-Verbindung für Mangansulfat in folgende Reaktionsgleichung angegeben:

MnSO₄ + C₆H₁₄N₂O₂ → [MnC₆H₁₃N₂O₂]HSO₄

Die entsprechende Reaktionsgleichung zur Herstellung von Eisen-Mono-Lysinat lautet:

FeSO₄ + C₆H₁₄N₂O₂ → [FeC₆H₁₃N₂O₂]HSO₄

Verallgemeinert lautet die Formel:

MA + C₆H₁₄N₂O₂ → [MC₆H₁₃N₂O₂]HA

Hierbei steht M für das Metall des Metallsalzes und A für den nichtmetallischen Teil des Metallsalzes. Berücksichtigt man die Ladungsverhältnisse im Metallsalz, steht M für das Metallkation, A für das entsprechende Anion und wobei Metallkation und Anion denen in Anspruch 1 spezifizierten Elementen entsprechen

Die Monolysinat-Verbindung, die nach einer Ausführungsform aus dieser Reaktionsmischung hervorgeht, nämlich [MnC₆H₁₃N₂O₂]HSO₄, besteht zu einem Gewichtsanteil von 17,38 % aus Mangan, zu 30,70 % aus Sulfat und zu 46,22 % aus Lysin. Das Endprodukt, der Mangan-Lysinat-Komplex, beinhaltet etwa 5 % bis maximal 10 % (Gewichtsprozent) Wasser. Im Endprodukt beträgt das Verhältnis von Gewichtsprozent Lysin zu Gewichtsprozent Mangan 1:2,56.

Die Monolysinat-Verbindung, die nach einer Reaktionsmischung gemäß einer anderen Ausführungsform der Erfindung hervorgeht, nämlich [FeC₆H₁₃N₂O₂]HSO₄, besteht zu einem Gewichtsanteil von 17,60 % aus Eisen, zu 30,61 % aus Sulfat und zu 46,10 % aus Lysin. Das Endprodukt, der Eisen-Lysinat-Komplex, beinhaltet etwa 5 % bis maximal 10 % (Gewichtsprozent) Wasser. Im Endprodukt beträgt das Verhältnis von Gewichtsprozent Lysin zu Gewichtsprozent Eisen 1:2,619.

Bevorzugte Mengenverhältnisse von Metallsulfat und Lysinlösung gemäß Ausführungsformen der Erfindung lassen sich aus der untenstehenden Tabelle für mehrere unterschiedliche Metallsulfate berechnen und hängt von der Konzentration der verwendeten Lysinlösung ab. Die Lysinlösung kann z.B. einen Lysin-Gewichtsanteil von 50 % haben. Die Mengen der Lysinlösung und des Metallsalzes werden vorzugsweise so gewählt, dass ein equimolares Verhältnis von Lysinmolekülen:Metallatomen des Metallsalzes (im Folgenden in den Tabellen mit "equimolarer Mischung bezeichnet) in der fertigen Reaktionslösung vorliegt. Es ist möglich, Lysin und Metallsalz in anderen molaren Verhältnissen in Lösung zu bringen um die Reaktionsmischung herzustellen, z.B. anstatt eines molaren Verhältnisses von 1:1 in einem molaren Verhältnis von 1:1,20 oder 1:0,80. Vorzugsweise werden die Reaktionsprodukte jedoch in untenstehendem Verhältnis in Lösung gebracht, da dadurch, wenn überhaupt, nur ein sehr kleiner stöchiometrischer Rest an Edukten in der Reaktionslösung verbleibt.

| **Metallsulfat** | **:** | **FLysin: "flüssig Lysin": Wasser mit 50% Gewichtsanteil Lysin** | **Metallsulfat [g/mol]** | **:** | **FLysin [g/mol]** | **GewichtsVerhältnis bei equimolarer Mischung** | | |
|---|---|---|---|---|---|---|---|---|
| Mangansulfat | : | FLysin | 151,00 | : | 290,19 | 1 | : | 1,92 |
| Eisenhydrid Tetrahydrat | : | FLysin | 198,3 | : | 290,19 | 1 | : | 1,46 |
| Eisenarbonat | : | FLysin | 115,85 | : | 290,19 | 1 | : | 2,50 |
| Eisensulfat | : | FLysin | 151,91 | : | 290,19 | 1 | : | 1,91 |
| Zinksulfat | : | FLysin | 161,45 | : | 290,19 | 1 | : | 1,80 |
| Magnesium-sulfat | : | FLysin | 120,37 | : | 290,19 | 1 | : | 2,41 |
| Calciumsulfat | : | FLysin | 136,11 | : | 290,19 | 1 | : | 2,13 |
| Natriumsulfat | : | FLysin | 142,04 | : | 290,19 | 1 | : | 2,04 |
| Nickelsulfat | : | FLysin | 154.76 | : | 290,19 | 1 | : | 1,88 |
| Kaliumchlorid | | FLysin | 74,55 | | 290,19 | 1 | : | 3,89 |
| Kaliumsulfat | : | FLysin | 174,26 | : | 290,19 | 1 | : | 1,83 |

Das Molekulargewicht von 290.19 g/Mol ist ein für eine 50%ige Lysinlösung errechneter Wert, der wie folgt berechnet wird: 1000 g einer reinen wässrigen 50%igen Lysinlösung beinhalten 500 g Lysin, welches ein Molekulargewicht von 146.19 g/mol hat, sowie 500 g Wasser, welches ein Molekulargewicht von 18,015 g/mol hat. In 1000 g Flüssiglysin ("FLysin") sind demnach 500g/ 146.19 g/mol = 3.42 mol Lysin und 500g/18,015 g/mol = 27,7 mol enthalten. Das molare Verhältnis von Lysin:H2O ist also 3.42 mol: 27,7 mol = 1:8,11. Demnach kommen in dieser 50%igen Lysinlösung auf ein Mol Lysin etwa 8 Mol Wasser. Das hypothetisch errechnete "Molekulargewicht einer 50%igen wässrigen Lysinlösung ist demnach 146.19 g/mol + 8,11 × 18,015 g/mol = 292,3 g/mol. Die obige Tabelle gibt einen rechnerisch gerundeten Wert von 290,19 g/mol an, der sich ergibt, wenn man von 8 mol H2O pro Mol Lysin mit einem Molekulargewicht von Wasser von rund 18 g/mol ausgeht. Wird eine Lysinlösung anderer Konzentration verwendet, sind ist das errechnete, "hypothetische" Molekulargewicht von FLysin in der obigen Tabelle entsprechend anzupassen.

Um Mangansulfatmonolysinat herzustellen, werden also 1 Gewichtsteil der oben genannten 50%igen Lysinlösung mit 1,92 Teilen Mangansulfat kombiniert, indem z.B. diese 1,92 Teile Mangansulfat in dem einen Teil der Lysinlösung in Lösung gebracht werden. In analoger Weise werden die Metallsalzmengen für andere Metallsalze berechnet.

Nach weiteren Ausführungsformen werden verschiedene Magnesiumsalze zur Herstellung eines Magnesiumsalz-Monolysinats verwendet. Die untenstehende Tabelle gibt Gewichtsverhätnisse für die Herstellung verschiedener Magnesiumlysinate an:

| **MAGNESIUMSALZ** | **:** | **FLysin: "flüssig Lysin": Wasser mit 50% Ge-wichtsanteil Lysin** | **MAGNE-SIUMSALZ [G/MOL]** | | **FLysin [g/mol]** | **Gewichts-Verhältnis bei equimolarer Mi-schung** | | |
|---|---|---|---|---|---|---|---|---|
| Magnesium-sulfat | : | FLysin | 120,37 | : | 290,19 | 1 | : | 2,41 |
| Magnesium-sulfat Heptahydrat | : | FLysin | 246,48 | : | 290,19 | 1 | : | 1,18 |
| Magnesium-carbonat | : | FLysin | 84,31 | : | 290,19 | 1 | : | 3,44 |
| Magnesiumcarbonat Monohyd-rat | : | FLysin | 102,32 | : | 290,19 | 1 | : | 2,84 |
| Magnesium-carbonat Dihydrat | : | FLysin | 120,34 | : | 290,19 | 1 | : | 2,41 |
| Magnesium-carbonat Trihydrat | . | FLysin | 138,35 | : | 290,19 | 1 | : | 2,10 |
| Magnesiumcarbonat Pentahyd-rat | : | FLysin | 210,40 | : | 290,19 | 1 | : | 1,38 |
| Magnesium-hydroxid | : | FLysin | 58,33 | : | 290,19 | 1 | : | 4,97 |
| Magnesium-chlorid | : | FLysin | 95,21 | : | 290,19 | 1 | : | 3,05 |
| Magnesium-chlorid Hexaydrat | : | FLysin | 203,30 | : | 290,19 | 1 | : | 1,43 |

Obiger Tabelle kann entnommen werden, dass manche Metallsalze als Hydrate vorliegen. In diesem Fall muss der Hydratwasseranteil bei der Berechnung des Gewichts bzw. der Menge des in Lösung zu bringenden Metallsalzes entsprechend berücksichtigt werden. Der Einfluss von Hydratwasser auf die Mischungsverhältnisse des FLysins und des jeweiligen Metallsalzes ist beispielhaft in der obenstehenden Tabelle für Magnesiumsalze angegeben. Untenstehende Tabelle enthält entsperchende Gewichts-Verhältnisse für Calciumsalze.

| **Calciumsalz** | **:** | **FLYSIN: "FLÜSSIG LYSIN": WASSER MIT 50% GEWICHTSANTEIL LYSIN** | **Calciumsalz [g/mol** | | **FLYSIN [G/MOL]** | **GEWICHTS-VERHÄLT-NIS BEI EQUIMOLARER MISCHUNG** | | |
|---|---|---|---|---|---|---|---|---|
| Calciumsulfat | : | FLysin | 136,11 | : | 290,19 | 1 | : | 2,13 |
| Calciumsulfat Hemihydrat | : | FLysin | 145,15 | : | 290,19 | 1 | : | 2,00 |
| Calciumsulfat Dihydrat | : | FLysin | 172,17 | : | 290,19 | 1 | : | 1,69 |
| Calciumsulfat Hydrat | : | FLysin | 154,16 | : | 290,19 | 1 | : | 1,88 |
| Calciumcarbonat | : | FLysin | 100,09 | : | 290,19 | 1 | : | 2,90 |
| Calciumhydroxid | : | FLysin | 74,10 | : | 290,19 | 1 | : | 3,92 |
| Calciumchlorid | : | FLysin | 110,98 | : | 290,19 | 1 | : | 2,61 |
| Calciumchlorid Dihydrat | : | FLysin | 147,02 | : | 290,19 | 1 | : | 1,97 |
| Calciumhydrid Tretrahydrat | : | FLysin | 183,04 | : | 290,19 | 1 | : | 1,59 |
| Calciumhydrid Hexahydrat | : | FLysin | 219,08 | : | 290,19 | 1 | : | 1,32 |

Um Bormonolysinate herzustellen, kann beispielsweise Borcarbonat oder Borsulfat als das Metallsalz verwendet werden. Um Natrium-Monolysinate zu erhalten, kann neben Natriumsulfat beispielsweise auch Natriumcarbonat oder Natriumcarbonat Monohydrat oder Natriumcarbonat Decahydrat als Metallsalz verwendet werden. Die obenstehenden Tabellen sind also nur exemplarisch zu verstehen.

Nach vollständiger Umsetzung der Edukte in die Monolysinat-Verbindung oder nach Erreichen des chemischen Reaktionsgleichgewicht des erfolgt in einem weiteren Schritt 608 eine Trocknung der flüssigen Reaktionsmischung zur Gewinnung des darin enthaltenen Reaktionsprodukt, der Monolysinat-Verbindung.

Beispielsweise kann die Trocknung mittels Sprühtrocknung (auch Zerstäubungstrocknung) durchgeführt werden. Bei der Sprühtrocknung wird mittels eines Zerstäubers die ausreagierte bzw. im Gleichgewicht befindliche Reaktionsmischung in einen Heißgasstrom eingebracht, der das in der Mischung enthaltene Reaktionsprodukt in sehr kurzer Zeit (wenige Sekunden bis Bruchteile einer Sekunde) zu einem feinen Pulver trocknet. Beispielsweise wird mittels eines Druckzerstäubers (bei je nach Bauart typischerweise 50 bis 250 bar), eines pneumatischen Zerstäubers (bei je nach Bauart typischerweise 1 bis 10 bar) oder eines Rotationszerstäubers (bei je nach Bauart typischerweise 4.000 bis 50.000 1/min) die zu trocknende Reaktionsmischung zerstäubt. Dadurch wird die Gesamtoberfläche der Flüssigkeit enorm vergrößert. Die zerstäubte Reaktionsmischung wird in einen Heißgasstrom eingesprüht, wodurch aufgrund der großen Oberfläche in sehr kurzer Zeit die Flüssigkeit verdampft und das feuchte Gut zu einem feinen Pulver trocknet. Da die Energie für die Verdampfung durch das Heißgas zur Verfügung gestellt wird, handelt es sich bei der Sprühtrocknung um eine sogenannte Konvektionstrocknung. Beim Heißgas handelt es sich vorzugsweise um Luft. Der Einsatz von Inertgasen ist aber auch möglich.

Die Eintrittstemperatur des Heißgases liegt im Bereich von 150-200°C, insb. Im Bereich von 170-190°C. Die Speisetemperatur der ausreagierten Reaktionsmischung, einer dunkelbraunen Lösung, liegt vorzugsweise im Bereich von 60-80°C. Der Sprühdruck liegt vorzugsweise im Bereich von 2,0 bis 3 bar, insb. 2,0 bis 2,8 bar. Der Feststoffgehalt der versprühten ausreagierten Reaktionsmischung liegt vorzugsweise bei ca. 40-60%, insbesondere bei ca. 45-52%.

Das anfallende pulverförmige Trocknungsgut kann nun abgeschieden und gesammelt werden. Beispielsweise kann ein Zyklonabscheider das durch Trocknung entstandenen Monolysinat-Pulver vom Luftstrom trennen. Der Sprühtrockner kann sowohl kontinuierlich als auch diskontinuierlich betrieben werden.

Die mittels Sprühtrocknung gewonnenen Monolysinat-Pulverpartikel haben typischerweise einen Durchmesser zwischen 80 µm und 100 µm. Nach einer Ausführungsform haben über 90%, vorzugsweise über 95% der Monolysinat-Pulverpartikel einen Durchmesser zwischen 80 µm und 100 µm.

Nach manchen Ausführungsformen der Erfindung werden die getrockneten Monolysinat-Pulverpartikel zur Verbesserung der Pulvereigenschaften (z. B. Pulverfließfähigkeit, Untersinkverhalten, Staubneigung) zu Granulaten agglomeriert. So wird beispielsweise sehr feines Monolysinat-Pulver in den Bereich der Zerstäuber zurückgeführt um dort Agglomeration zu fördern.

In manchen Ausführungsformen des Verfahrens wird der Schritt der Trocknung der ausreagierten Reaktionsmischung in einem eigenen Schritt durchgeführt, welcher vor dem Schritt der Erzeugung eines Granulats aus dem beim Trocknen gewonnenen Pulver erfolgt.

In anderen Ausführungsformen erfolgt die Erzeugung des Monolysinat-Granulats im Zuge der Trocknung. Ein Beispiel für diese Verfahrensvariante ist die Sprühgranulation, bei welcher zunächst, wie bei einer reinen Sprühtrocknung, trockene Kleinstpartikel in einem Verarbeitungsgefäß ("Wirbelbett") im Schwebezustand gehalten werden. Die Oberfläche dieser Kleinstpartikel dient als Kristallisationskern für weitere durch die Vernebelung generierte kleine Tropfen. Im Verfahren der Sprühgranulation erfolgt somit eine Trocknung und Granulation in einem gemeinsamen Verfahrensschritt, der eine Kontrolle des Partikelwachstums und damit auch eine Kontrolle der Partikelgröße und teilweise auch von deren Oberflächenstruktur ermöglicht.

Ein "Granulat" im Sinne diese Erfindung ist eine partikelförmige Substanz, wobei der Durchmesser von mindestens 95%, vorzugsweise mindestens 97% der Partikel im Bereich zwischen 100µm und 800µm liegt. Das Granulat hat vorzugsweise ein Schüttgewicht von 700-800 g/Liter (Eisen-Monolysinat: ca. 750 g/l, Mangan-Monolysinat: ca. 760-770 g/L) bei einer Restfeuchte von unter 5%, z.B. einer Restfeuchte von 2-3%.

Die Verarbeitung des Monolysinat-Pulvers zu einem Granulat hat mehrere Vorteile wie zum Beispiel die verringerte Staubneigung, eine bessere Handhabbarkeit, eine bessere Rieselfähigkeit, eine reduzierte Neigung zur Klumpenbildung und den Vorteil der einfacheren Dosierung, zumindest in Ausführungsformen, gemäß welchen das Granulat mit Füll -oder Zusatzstoffen "gestreckt" wird um dadurch eine geringere Konzentration der Monolysinat-Verbindung pro Volumeneinheit zu erzielen.

Bei manchen Anwendungen ist es jedoch vorteilhaft, das getrocknete Monolysinat-Pulver direkt zu vertreiben und weiterzuverarbeiten, denn aufgrund der großen spezifischen Oberfläche des Pulvers löst sich das sprühgetrocknete Pulver schneller in Wasser als das entsprechende Granulat.

Typischerweise enthält die Monolysinat-Verbindung, die mit dem hier beschriebenen Verfahren erzeugt wird, Kristallwasser. Beispielsweise beinhaltet die Monolysinat-Verbindung [MnC₆H₁₃N₂O₂]HSO₄ etwa 5 % ihres Gewichts an Kristallwasser.

Die gemäß des hier beschriebenen verfahrensgewonnene Monolysinat-Verbindung hat den Vorteil, dass sie besonders rein ist, d. h. weitgehend frei ist von Verunreinigungen, die entweder bereits in den Ausgangsstoffen enthalten sind oder erst im Zuge der Verarbeitung eingebracht werden. Insbesondere wird der Eintrag von Schwermetallen aus korrodierten Stahlbehältern vermieden. Das Verfahren ist kostengünstig und in kurzer Zeit durchzuführen, da auf bereits im Handel erhältliche wässrige Lysinlösungen zurückgegriffen werden kann.

Die so gewonnene Monolysinat-Verbindung kann auf vielfältige Weise zum Einsatz kommen: Lysin ist eine der limitierenden Aminosäuren und dient der Synthese von Nukleinsäuren, dem Kohlenhydratstoffwechsel und ist wichtig für die Produktion von Antikörpern, Hormonen und Enzymen. Lysin verbessert bei vielen Organismen, insbesondere auch Nutztieren, den Stickstoffhaushalt, verstärkt die Sekretion von Verdauungsenzymen, befördert den Kalziumstransport der Zellen, und führt ganz allgemein zu einem besseren Gesundheitszustand, zu einer besseren Nahrungsverwertung und verbesserten Leistungseigenschaften. Die physiologische Wirksamkeit der dem Futtermittel zugegebenen Spurenelemente lässt sich durch Einbindung dieser Spurenelemente (Metalle) in die Monolysinat-Verbindung erhöhen, sodass insgesamt weniger Metallsalze bzw. Metallverbindungen dem Futter zugesetzt werden müssen, wodurch auch die Eintragung entsprechender Metalle über Ausscheidungen der Tiere in Flüsse und Felder reduziert wird.

Ein Einsatzgebiet der hier gemäß Ausführungsformen der Erfindung beschriebenen Monolysinat-Verbindung ist also ihre Verwendung als Komponente von Tierfutter, zum Beispiel als Bestandteil einer Spurenelementmischung, die als Nahrungsergänzungsmittel bzw. Futterergänzungsmittel für Nutz- und Heimtiere dient.

Außerdem kann die Monolysinat-Verbindung als Bestandteil von Pflanzendünger verwendet werden. In der Pflanzendüngung wurden mehrere positive Effekte der hier beschriebenen Monolysinat-Verbindung beobachtet, unter anderem eine Steigerung der Blattabsorption von Spurenelementen wie Eisen, Mangan, Zink, Calcium und Magnesium. Das Lysin, das in Form der Monolysinat-Verbindung von einer Pflanze aufgenommen wird, stärkt das Immunsystem der Pflanze und stimuliert die Chlorophyllsynthese.

**Figur 11** zeigt eine ein Flussdiagramm eines verallgemeinerten Verfahrens zur Herstellung einer Monolysinat-Verbindung 200, 300, 400, 500, 600, 700, 800, 900 wie z.B. in Figuren 2-9 dargestellt. Im Schritt 702 wird die bereits in der Beschreibung der Figur 10 erwähne flüssige Reaktionsmischung erzeugt, wobei offen bleibt, ob, wie in Figur 10 gezeigt, zunächst eine flüssige Lysinlösung bereitgestellt wird, in welcher dann das Metallsalz in Lösung gebracht wird, oder ob zuerst Lysin in einer wässrigen Lösung, in der das Metallsalz bereits in der gewünschten Menge gelöst ist, in Lösung gebracht wird, oder ob Lysin und Metallsalz gleichzeitig in Wasser in Lösung gebracht werden. All diese Varianten können angewandt werden, um zu der flüssigen Reaktionsmischung zu gelangen. Vorzugsweise erfolgt das In-Lösung-Bringen von Lysin und/oder Metallsalz bei erhöhten Temperaturen von mindestens 30°C, da dies den Lösungsprozess beschleunigt. Der Schritt 704 der chemischen Umsetzung der gelösten Edukte in das Monolysinat erfolgt, wie bereits beschrieben, vorzugsweise bei Temperaturen im Bereich von 60°C-90°C unter ständigem Rühren während einer Dauer von typischerweise 20-60 Minuten, z.B. 25-35 Minuten. Danach kann die ausreagierte Lösung zur Gewinnung des Monolysinats getrocknet 706 und ggf. auch granuliert werden.

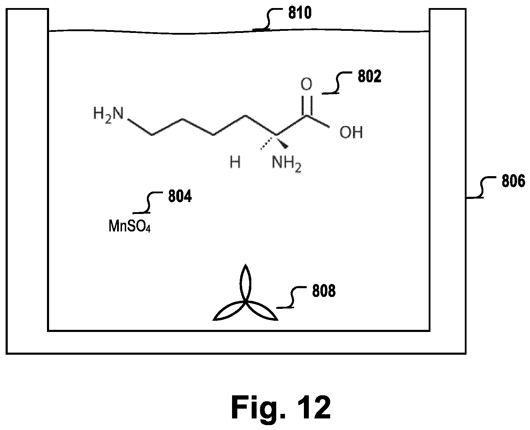

**Figur 12** zeigt eine schematische Darstellung eines Behältnisses 806, welches eine flüssige Reaktionsmischung 810 beinhaltet, die etwa 30 Minuten lang bei einer Temperatur zwischen 60 und 90 °C gerührt wird, um die in ihr gelösten Substanzen zur Reaktion zu bringen. Bei der Reaktionsmischung 810 handelt es sich um Wasser, in welchem zum einen Lysin 802 und zum anderen ein Metallsalz, zum Beispiel Mangansulfat 804 gelöst sind. Im Zuge des Lösungsprozesses dissoziiert das Metallsalz in positiv geladene Metallionen sowie negativ geladene Anionen, zum Beispiel Sulfationen. Diese Edukte werden im Zuge der in der Reaktionsmischung ablaufenden chemischen Reaktion zu einer Monolysinat-Verbindung 200, 300, 400, 500, 600, 700, 800, 900 umgesetzt, wie diese zum Beispiel in Figuren 2-9 dargestellt ist. Bei dem Reaktionsbehälter 806 kann es sich zum Beispiel um einen Stallbehälter mit einer Rühr- oder sonstigen Mischvorrichtung 808 handeln.

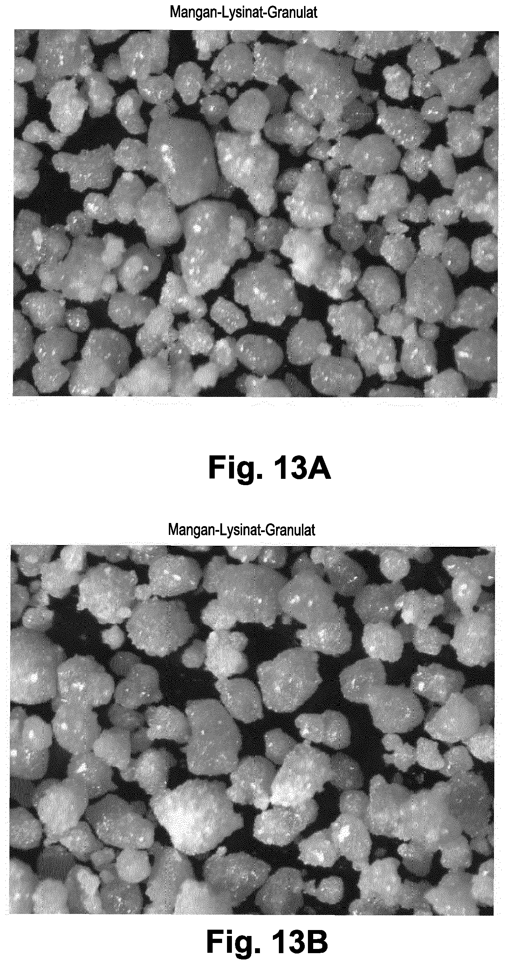

**Figuren 13A und 13B** zeigen Fotos eines durch Sprühgranulation erhaltenen Granulats einer Mangan-Monolysinat-Verbindung gemäß der Formel [MnC₆H₁₃N₂O₂]HSO₄, wobei es sich bei der Mangan-Monolysinat-Verbindung um ein Hydrat handelt.

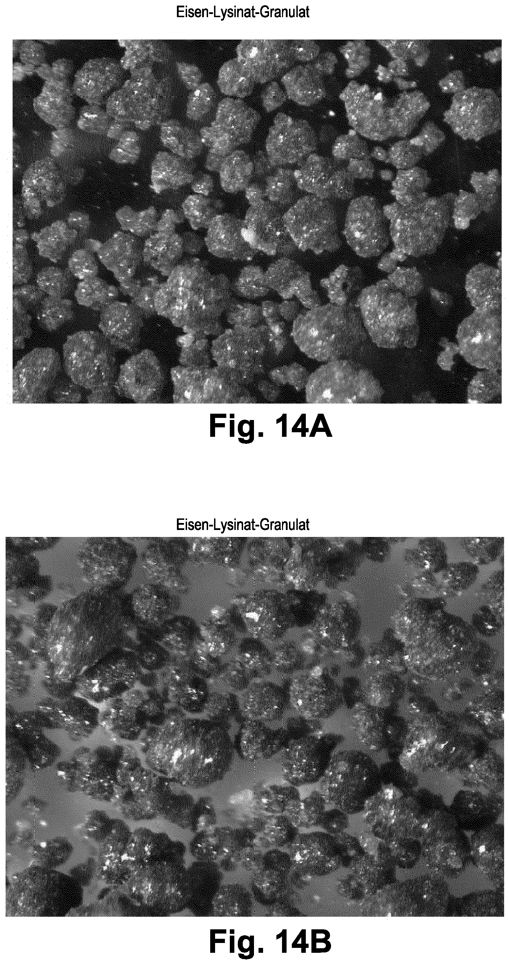

**Figuren 14A und 14B** zeigen Fotos eines durch Sprühgranulation erhaltenen Granulats einer Eisen-Monolysinat-Verbindung gemäß der Formel [FeC₆H₁₃N₂O₂]HSO₄, wobei es sich bei der Eisen-Monolysinat-Verbindung um ein Hydrat handelt.

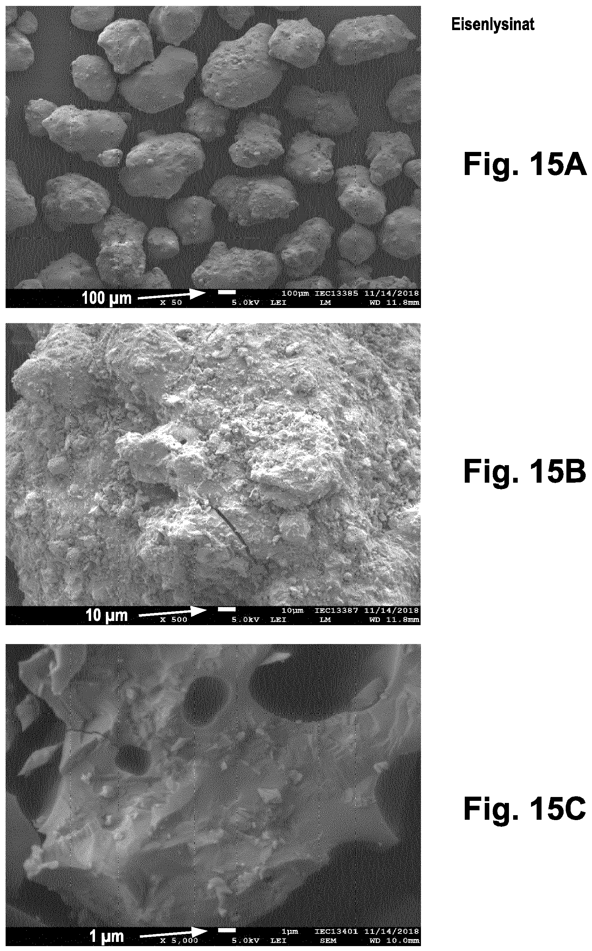

**Figur 15** zeigt elektronenmikroskopische Aufnahmen von Eisen-Monolysinat-Sulfat Granulatkörnern bei verschiedenen Auflösungen. Figur 15A enthält in der schwarzen Bildleiste einen weißen horizontalen Balken, dessen Länge 100 µm entspricht. Die in diesem Bild dargestellte Granulatstruktur hängt stark vom jeweils geäwhlten Trocknungs- bwz. Granulationsprozess ab. Der entsprechende Balken in Figur 15B entspricht 10 µm, der in Figur 15C 100µm. Insbesondere in Figur 15C ist die Oberfläche der Kristallstruktur des Eisen-Monolysinat-Sulfat-Salzes, die eher unabhängig ist vom gewählten Trocknungs- oder Granulationsverfahren, gut erkennbar. Die Oberfläche weißt zahlreiche Krater und runde Aussparungen auf, aber auch scharfe und eher linienförmige Bruchkanten.

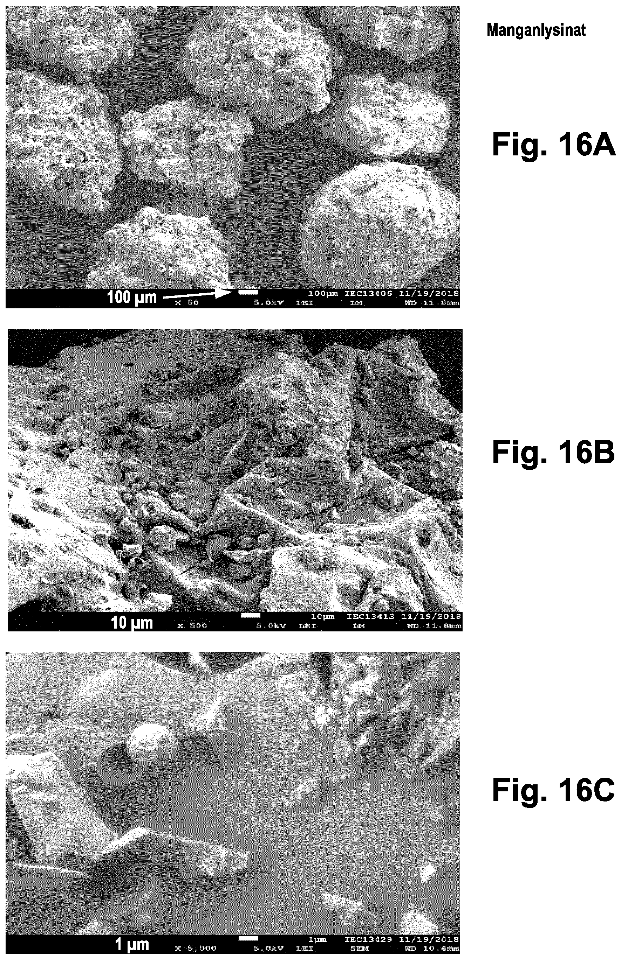

**Figur 16** zeigt elektronenmikroskopische Aufnahmen von Mangan-Monolysinat-Sulfat Granulatkörnern bei verschiedenen Auflösungen. Figur 16A enthält in der schwarzen Bildleiste einen weißen horizontalen Balken, dessen Länge 100 µm entspricht. Der entsprechende Balken in Figur 16B entspricht 10 µm, der in Figur 16C 100µm. Insbesondere in Figur 16C ist die Oberfläche der Kristallstruktur des Mangan-Monolysinat-Sulfat-Salzes gut erkennbar. Die Oberfläche weißt zahlreiche Krater und runde Aussparungen auf, aber auch scharfe und eher linienförmige Bruchkanten. Insgesamt wirkt die Oberfläche etwas glatter als die Oberfläche des Eisenlysinat-Salzes in Figur 15.

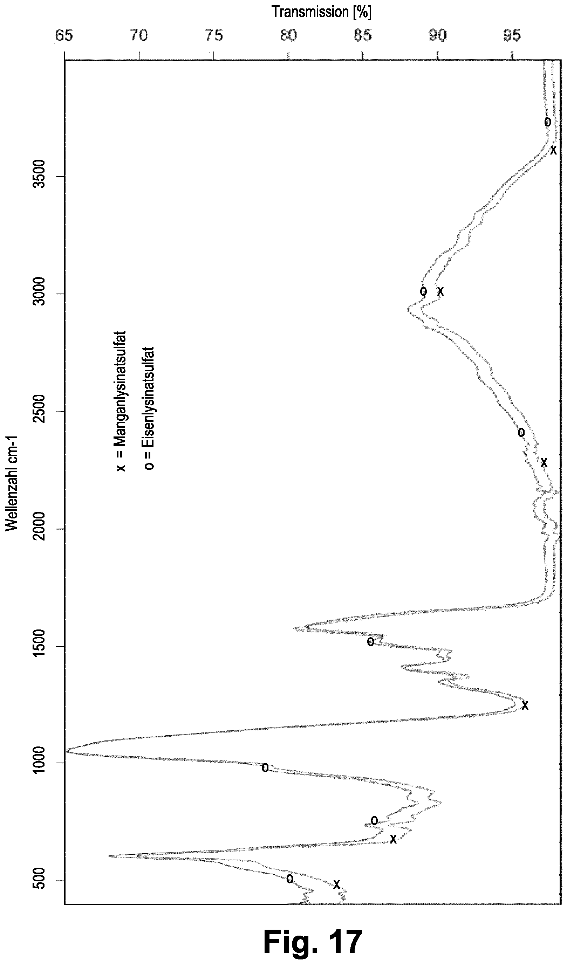

**Figur 17** zeigt Ergebnisse einer IR-Analytik von Eisen- bzw. Manganmonolysinat. Die untenstehende Tabelle beinhaltet stöchiometrische Aspekte dieser Verbindungen auf Basis von Elementanalytik der entsprechenden Manganverbindung. Von Eisenlysinatsulfat und Manganlysinatsulfat, die mittels einer Ausführungsform des erfindungsgemäßen Verfahrens hergestellt wurden, wurden zur Charakterisierung der Zusammensetzung Infrarot- Spektren in ATR-IR - Technik aufgenommen. Diese Vorgehensweise vermeidet jegliche Probenvorbereitung und damit eventuell einhergehende unerwünschte Veränderung der Analyte. Beide IR-Spektren (Manganlysinatsulfatspektrum an mehreren Stellen mit einem "x" gekennzeichnet, Eisenlysinatsulfatspektrum an mehreren Stellen mit einem Kreis gekennzeichnet), werden dominiert von einer Bande im Bereich von 1050 Wellenzahlen, die typisch für IR-Schwingungen der Atome Sauerstoff und Schwefel innerhalb eines Sulfatrestes ist. Diese Bande ist bei diesen beiden Metallverbindungen nahezu deckungsgleich. Im Bereich um 1580 cm-1 zeigt sich die erwartete, charakteristische Kohlenstoff-Sauerstoff-Streckschwingung γ_{C=O} in Form der Carboxylat-Bande der eingesetzten Aminosäure Lysin. Im Falle der Eisenverbindung ist diese Schwingung auf einen Wert von 1582 Wellenzahlen verschoben, während sie im Mangan-Pendant bei 1575 cm-1 erscheint. Zusätzlich finden sich ausgeprägte Banden knapp unterhalb von 3000 Wellenzahlen, die typisch für Streckschwingungen γ_{C-H} C sind und hier insbesondere den vier Methyleneinheiten des Lysins zuzuordnen sind. Bereits aus der Infrarot-Spektroskopie ergibt sich somit jeweils ein Metalllysinatsulfat als tatsächlich vorliegendes Produkt. Das Metall selbst kann mittels gezielter Metallgehaltsanalyse (->LUFA) bestimmt werden. Im Gegensatz zu ähnlichen bekannten Verbindungen mit Chelat-Charakter fehlen bei beiden Produkten die typischen, weit in den hochenergetischen Bereich um 3500 cm-1 verschobenen Banden, so dass hier kein "Chelat" im eigentlichen Wortsinne - mit koordinativer Bindung der α-Aminogruppe zum Metallkation - vorliegt, sondern stattdessen klassische Salze der Aminosäure mit den Metallen gebildet wurden mit ionischem Charakter. Außerdem wurde für die Manganverbindung getestet, ob sich die stöchiometrische Zusammensetzung, die sich rechnerisch aus der detaillierten Elementanalyse (nicht nur C,H- bzw. C,H,N-Analyse mit standardmäßiger Bestimmung der Elemente Kohlenstoff, Sauerstoff und Stickstoff, sondern im konkreten Fall unter Hinzuziehen der Gehalte an Schwefel und Sauerstoff) ergibt, übereinstimmt mit den zu erwartenden Werten. Das Ergebnis ist der unten aufgeführten Tabelle zu entnehmen.

| Element | Gemessene Menge [Massen%] | Rechnerisch erwartete Menge für Mangangsulfatmonolysinat (wasserfrei), C6H14MnN2O6S, 297,18 g/mol [Massen%] | Rechnerisch erwartete Menge für Mangangsulfatmonolysinat C6H16MnN2O7S, 315,20 g/mol (Monohydrat) [Massen%] |
|---|---|---|---|
| C | 22,68 | 24,25 | 22,86 |
| H | 5,29 | 4,75 | 5,12 |
| Mn | 16,6 | 18,49 | 17,43 |
| N | 8,97 | 9,43 | 8,89 |
| O | 31,45 | 32,30 | 35,53 |
| S | 10,55 | 10,97 | 10,17 |
| | | | |
| Freies Wasser | 4,1 | | |
| Gesamtsumme | 99,64 % | 100% | 100% |

Aus den gemessenen Massenprozentanteilen der quantitativ bestimmten Elemente Kohlenstoff, Wasserstoff, Stickstoff, Sauerstoff und Schwefel (CHNOS-Analyse) ergibt sich im Zusammenspiel mit der LUFA-Metallanalytik, dass tatsächlich auf ein Mangansulfatmonolysinat (synonym: Manganmonolysinatsulfat) zu schließen ist.

Dieses liegt allerdings nicht wasserfrei vor, sondern als Monohydrat mit einem Äquivalent Kristallwasser (siehe rechte Spalte der Tabelle).

Aus dem stöchiometrischen Verhältnis, dass jeweils ein zweiwertiges Metallkation mit einem Lysinatanion und einem Sulfat eine Grundeinheit bildet, ist nicht zwingend auf den monomeren Charakter, d.h. auf eine 1:1:1 - Verbindung (ggf. zuzüglich Kristallwasser) zu schließen. Stattdessen sind grundsätzlich auch oligomere Strukturen auf Basis mehrerer dieser Grundeinheiten denkbar, wie diese z.B. in Figur 8 dargestellt sind. Bei einem oligomeren Monolysinat können die Anionen des Metallsalzes, also z.B. die Sulfatreste, als Bindeglieder (also z.B. in Form von SO₄²⁻ Anionen) agieren, während bei einer monomeren Struktur eine Valenz des SO₄²⁻ Anions mit einem Wasserstoffatom bzw. Proton abgesättigt ist.

### Bezugszeichenliste

- 100: Strukturformel L-Lysin
- 102: Aminosäurerest der Aminosäure Lysin
- 104: Aminogruppe der Aminosäure Lysin
- 106: Carboxylgruppe der Aminosäure Lysin
- 200: allgemeine Strukturformel Monolysinat-Verbindung
- 300: Beispiel für Monolysinat gemäß Fig. 2
- 400: allgemeine Strukturformel Monolysinat-Verbindung
- 500: Beispiel für Monolysinat gemäß Fig. 4
- 600: allgemeine Strukturformel Monolysinat-Verbindung
- 602-608: Schritte
- 700: Beispiel für Monolysinat gemäß Fig. 6
- 702-706: Schritte
- 800: allgemeine Strukturformel Monolysinat-Verbindung
- 802: Lysinmolekül
- 804: Mangansulfat
- 806: Reaktionsverhältnis
- 808: Rührvorrichtung
- 810: flüssige Reaktionsmischung
- 900: Beispiel für Monolysinat gemäß Fig. 8

## Patentansprüche

1. Verfahren zur Herstellung einer Monolysinat-Verbindung, umfassend:
- Bereitstellung (302) einer wässrigen Lysinlösung;
- Erzeugung (304) einer flüssigen Reaktionsmischung (810) durch Lösen eines Metallsalzes (404) in der wässrigen Lysinlösung, wobei die flüssige Reaktionsmischung durch Lösen eines Metallsalzes in der wässrigen Lysinlösung in einem molaren Verhältnis von 1 Mol eines Metallatoms des Metallsalzes zu 1 Mol des Lysins erzeugt wird, wobei die wässrige Lysinlösung und die flüssige Reaktionsmischung im Wesentlichen frei sind von Chloriden und Cl⁻- Ionen, und insbesondere im Wesentlichen frei ist von Lysin-HCL Salzen;
- Bereitstellung (502) der flüssigen Reaktionsmischung (810), in welcher Lysin (802) und das Metallsalz (404) gelöst sind,;
- Umsetzung (306) des in der Reaktionsmischung gelösten Lysins (802) und des Metallsalzes in die Monolysinat-Verbindung;
- Trocknung (308) der flüssigen Reaktionsmischung zur Gewinnung der Monolysinat-Verbindung,
wobei es sich bei dem Metallsalz um ein Metallsulfat, ein Metallhydroxid oder ein Metallcarbonat handelt, wobei es sich bei dem Metall des Metallsalzes um ein zweiwertiges Metall, nämlich Mn²⁺, Fe²⁺, Zn²⁺, Ca²⁺, Mg²⁺, Co²⁺, Na²⁺, oder Ni²⁺ handelt, und wobei es sich bei der Monolysinat-Verbindung um eine Verbindung nach einer der folgenden Strukturformeln a), b), c) oder d) handelt, wobei M für ein metallisches Kation des Metallsalzes und A für das Anion des Metallsalzes steht:
a) 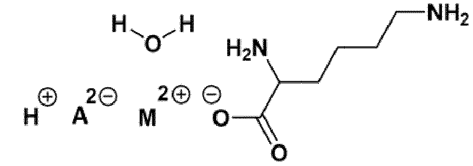
b) 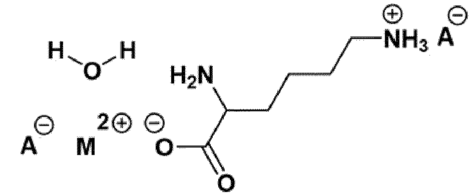
c) 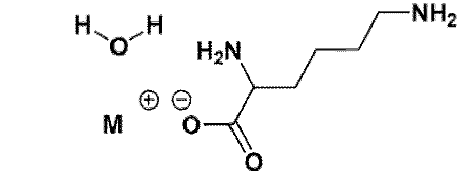
d)

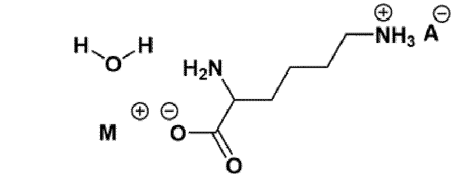

2. Verfahren nach einem der vorigen Ansprüche, wobei in der Monolysinat-Verbindung genau ein Lysinmolekül mit genau einem Metallatom des Metallsalzes verbunden ist, wobei es sich bei der Bindung um eine ionische Bindungen handelt.

3. Verfahren nach einem der vorigen Ansprüche, wobei es sich bei der Monolysinat-Verbindung um ein Mangan-Monolysinat-Sulfat oder um ein Einsen-Monolysinat-Sulfat handelt.

4. Verfahren nach einem der vorigen Ansprüche, wobei es sich bei dem Metallsalz um ein Zinksulfat (ZnSO₄), Eisensulfat (FeSO₄) oder Mangansulfat (MnSO₄) handelt.

5. Verfahren nach einem der vorigen Ansprüche, wobei die Umsetzung eine mechanische Durchmischung des gelösten Lysins und des gelösten Metallsalzes bei einer Temperatur von mindestens 60°C, vorzugsweise 60°C - 90°C für mindestens 15 Minuten umfasst.

6. Verfahren nach einem der vorigen Ansprüche, wobei die bereitgestellte wässrige Lysinlösung einen Lysinanteil von mindestens 30 Gewichtsprozent, vorzugsweise mindestens 40% Gewichtsprozent der wässrigen Lösung hat.

7. Verfahren nach einem der vorigen Ansprüche, ferner umfassend:
- Erzeugung eines Granulats aus der Monolysinat-Verbindung nach oder während der Trocknung.

8. Monolysinat-Verbindung mit der folgenden Strukturformel, wobei M für ein metallisches Kation eines Metallsalzes und A für das Anion des Metallsalzes steht gemäß einer der Formeln a), b), c) oder d):
a) 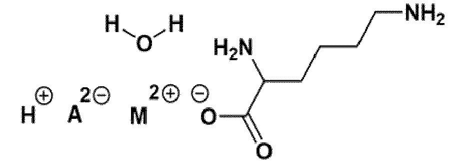
b) 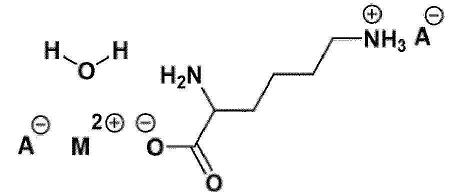
c) 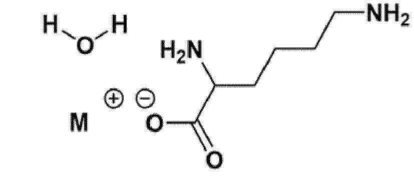
d) 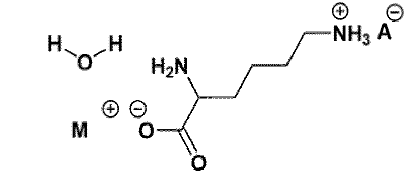
, wobei A ein Sulfatrest oder ein Hydroxidion oder ein Carbonatrest ist, und wobei es sich bei dem Metall des Metallsalzes um ein zweiwertiges Metall, nämlich Mn²⁺, Fe²⁺, Zn²⁺, Ca²⁺, Mg²⁺, Co²⁺, Na²⁺, oder Ni²⁺ handelt, wobei die Verbindung im Wesentlichen frei ist von Chloriden und Cl⁻ - Ionen.

9. Verwendung einer Monolysinat-Verbindung gemäß Anspruch 8 als Futtermittelzusatz für Nutz- und Haustiere und/oder als Gärzusatz und/oder als Düngemittelzusatz und/oder als Nahrungsmittelzusatz und/oder als Nahrungsergänzungsmittel.

## Claims

1. Method for preparing a monolysinate compound, comprising:
- providing (302) an aqueous lysine solution;
- producing (304) a liquid reaction mixture (810) by dissolving a metal salt (404) in the aqueous lysine solution, wherein the liquid reaction mixture is produced by dissolving a metal salt in the aqueous lysine solution in a molar ratio of 1 mol of a metal atom of the metal salt to 1 mol of the lysine, wherein the aqueous lysine solution and the liquid reaction mixture are substantially free of chlorides and Cl⁻ions, and in particular are substantially free of lysine-HCl salts;
- providing (502) the liquid reaction mixture (810) in which lysine (802) and the metal salt (404) are dissolved;
- reacting (306) the lysine (802) dissolved in the reaction mixture and the metal salt to form the monolysinate compound;
- drying (308) the liquid reaction mixture to obtain the monolysinate compound,
wherein the metal salt is a metal sulfate, a metal hydroxide, or a metal carbonate, wherein the metal of the metal salt is a divalent metal, in particular Mn²⁺, Fe²⁺, Zn²⁺, Ca²⁺, Mg²⁺, Co²⁺, Na²⁺, or Ni²⁺, and wherein the monolysinate compound is a compound according to any one of the following structural formulas a), b), c), or d), where M represents a metal cation of the metal salt and A represents the anion of the metal salt:
a) 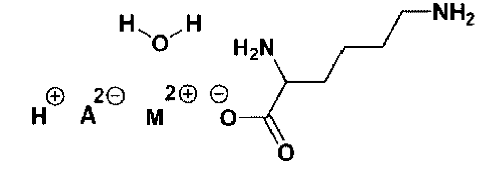
b) 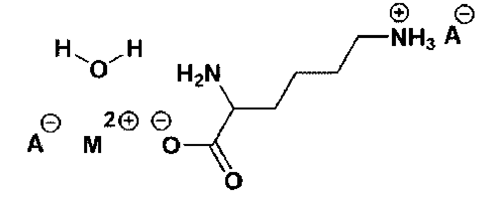
c) 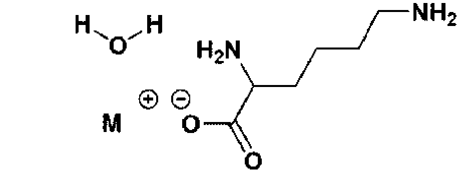
d)

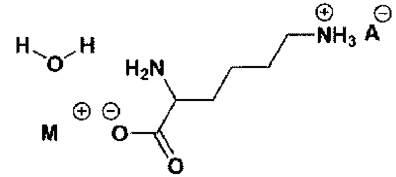

2. Method according to any one of the preceding claims, wherein in the monolysinate compound, exactly one lysine molecule is bonded to exactly one metal atom of the metal salt, wherein the bond is an ionic bond.

3. Method according to any one of the preceding claims, wherein the monolysinate compound is a manganese monolysinate sulfate or an iron monolysinate sulfate.

4. Method according to any one of the preceding claims, wherein the metal salt is a zinc sulfate (ZnSO₄), iron sulfate (FeSOa), or manganese sulfate (MnSO₄).

5. Method according to any one of the preceding claims, wherein the reaction comprises mechanically mixing the dissolved lysine and the dissolved metal salt at a temperature of at least 60°C, preferably 60°C-90°C, for at least 15 minutes.

6. Method according to any one of the preceding claims, wherein the provided aqueous lysine solution has a lysine content of at least 30% by weight, preferably at least 40% by weight, of the aqueous solution.

7. Method according to any one of the preceding claims, further comprising:
- producing a granulate from the monolysinate compound after or during the drying.

8. Monolysinate compound having the following structural formula, where M represents a metal cation of a metal salt and A represents the anion of the metal salt, according to any one of the formulas a), b), c), or d): 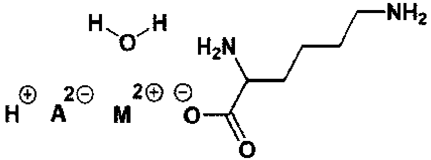
b) 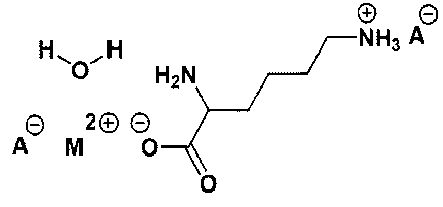
c) 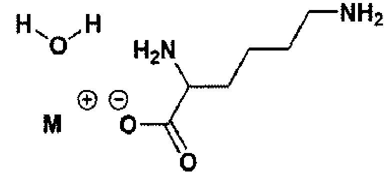
d) 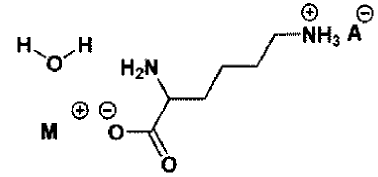
where A is a sulfate group or a hydroxide ion or a carbonate group, and wherein the metal of the metal salt is a divalent metal, in particular Mn²⁺, Fe²⁺, Zn²⁺, Ca²⁺, Mg²⁺, Co²⁺, Na²⁺, or Ni²⁺, wherein the compound is substantially free of chlorides and Cl⁻ ions.

9. Use of a monolysinate compound according to claim 8 as a feed additive for livestock and pets and/or as a fermentation additive and/or as a fertilizer additive and/or as a food additive and/or as a food supplement.

## Revendications

1. Procédé de fabrication d'un composé monolysinate comprenant :
- la préparation (302) d'une solution de lysine aqueuse ;
- l'élaboration (304) d'un mélange réactionnel (810) liquide par dissolution d'un sel de métal (404) dans une solution de lysine aqueuse, où le mélange réactionnel liquide est créé par dissolution d'un sel de métal dans la solution de lysine aqueuse dans un rapport molaire de 1 mole d'un atome de métal du sel de métal pour 1 mole de lysine, où la solution de lysine aqueuse et le mélange réactionnel liquide sont essentiellement exempts de chlorures et d'ions Cl⁻, et en particulier essentiellement exempts de sels lysine-HCl ;
- la préparation (502) du mélange réactionnel (810) liquide dans lequel la lysine(802) et le sel de métal (404) sont dissouts ;
- la conversion (306) de la lysine (802) solubilisée dans le mélange réactionnel et du sel de métal pour donner le composé monolysinate ;
- le séchage (308) du mélange réactionnel liquide pour l'obtention du composé monolysinate,
dans lequel, dans le cas du sel de métal, il s'agit d'un sulfate de métal, un hydroxyde de métal ou un carbonate de métal, où, dans le cas du métal du sel de métal, il s'agit d'un métal bivalent, à savoir Mn²⁺, Fe²⁺, Zn²⁺, Ca²⁺, Mg²⁺, Co²⁺, Na²⁺ ou Ni²⁺, et dans lequel, dans le cas du composé monolysinate, il s'agit d'un composé selon l'une des formules structurelles a), b), c) ou d) suivantes, où M représente un cation métallique du sel de métal et A représente l'anion du sel de métal :
a) 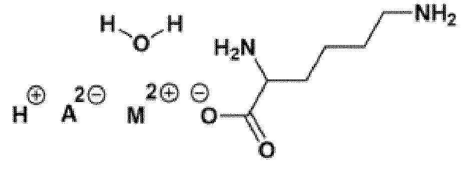
b) 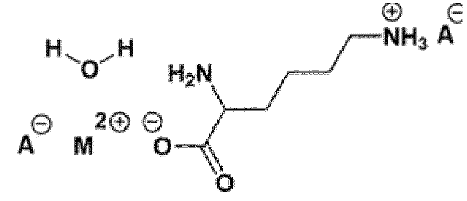
c) 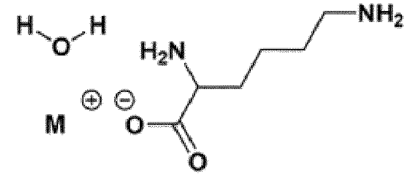
d)

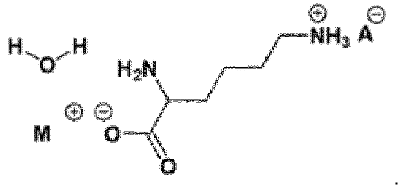

2. Procédé selon l'une des revendications précédentes, dans lequel, dans le composé monolysinate, précisément une molécule de lysine est reliée avec précisément un atome métallique du sel de métal, où, dans le cas de la liaison, il s'agit d'une liaison ionique.

3. Procédé selon l'une des revendications précédentes, dans lequel, dans le cas du composé monolysinate, il s'agit d'un sulfate monolysinate de manganèse ou d'un sulfate monolysinate de fer.

4. Procédé selon l'une des revendications précédentes, dans lequel, dans le cas du sel de métal, il s'agit d'un sulfate de zinc (ZnSO₄), d'un sulfate de fer (FeSOt) ou d'un sulfate de manganèse (MnSO₄).

5. Procédé selon l'une des revendications précédentes, dans lequel la conversion comprend un mélange mécanique de la lysine dissoute et du sel de métal dissout à une température d'au moins 60 °C, de préférence de 60 °C à 90 °c, pendant au moins 15 minutes.

6. Procédé selon l'une des revendications précédentes, dans lequel la solution de lysine aqueuse préparée possède une proportion de lysine d'au moins 30 pourcents en poids, de préférence d'au moins 40 % pourcents en poids de la solution aqueuse.

7. Procédé selon l'une des revendications précédentes, comprenant en outre :
- la création de granulés à partir du composé monolysinate après ou pendant le séchage.

8. Composé monolysinate avec les formules structurelles suivantes, dans lequel M représente un cation métallique d'un sel de métal et A représente l'anion du sel de métal, selon l'une des formules a), b), c) ou d) :
a) 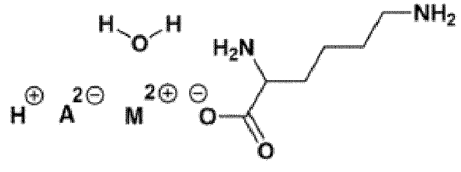
b) 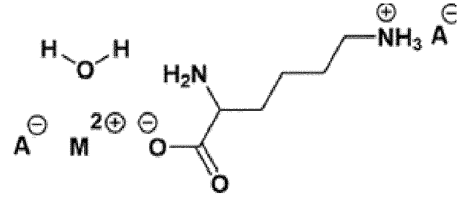
c) 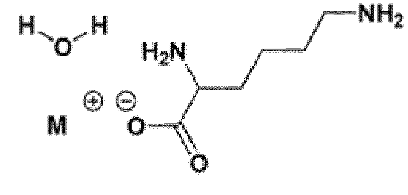
d) 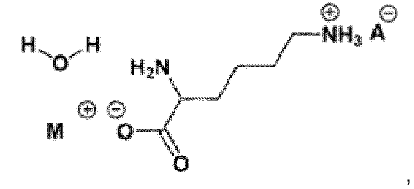
où A est un reste sulfate ou un ion hydroxyde ou un reste carbonate, et où, dans le cas du métal du sel de métal, il s'agit d'un métal bivalent, à savoir Mn²⁺, Fe²⁺, Zn²⁺, Ca²⁺, Mg²⁺, Co²⁺, Na²⁺ ou Ni²⁺, où le composé est essentiellement exempt de chlorures et d'ions Cl⁻.

9. Utilisation d'un composé monolysinate selon la revendication 8 en tant que complément alimentaire pour du bétail et des animaux domestiques, et/ou en tant qu'additif de fermentation, et/ou en tant qu'additif d'engrais, et/ou en tant qu'additif alimentaire, et/ou en tant que complément alimentaire.
